(19) Europäisches Patentamt
European Patent Office
Office européen des brevets

(11) EP 1 914 232 B1

(12) # EUROPEAN PATENT SPECIFICATION

(45) Date of publication and mention of the grant of the patent:
**15.12.2010 Bulletin 2010/50**

(21) Application number: **06781147.1**

(22) Date of filing: **11.07.2006**

(51) Int Cl.:
***C07D 401/04*** *(2006.01)* ***A61K 31/454*** *(2006.01)*
***A61P 3/04*** *(2006.01)* ***A61P 9/00*** *(2006.01)*
***A61P 9/02*** *(2006.01)* ***A61P 9/10*** *(2006.01)*
***A61P 13/02*** *(2006.01)* ***A61P 19/02*** *(2006.01)*
***A61P 19/06*** *(2006.01)* ***A61P 25/06*** *(2006.01)*
***A61P 25/14*** *(2006.01)* ***A61P 25/16*** *(2006.01)*
***A61P 25/18*** *(2006.01)* ***A61P 25/24*** *(2006.01)*
***A61P 25/28*** *(2006.01)* ***A61P 29/00*** *(2006.01)*
***A61P 43/00*** *(2006.01)*

(86) International application number:
**PCT/JP2006/314122**

(87) International publication number:
**WO 2007/007890 (18.01.2007 Gazette 2007/03)**

(54) **N-DIHYDROXYALKYL-SUBSTITUTED 2-OXOIMIDAZOLE DERIVATIVES**

N-DIHYDROXYALKYL-SUBSTITUTIERTE 2-OXOIMIDAZOLDERIVATE

DERIVES DU 2-OXOIMIDAZOLE A SUBSTITUTION N-DIHYDROXYALKYLE

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**HR**

(30) Priority: **13.07.2005 JP 2005204264**

(43) Date of publication of application:
**23.04.2008 Bulletin 2008/17**

(73) Proprietor: **BANYU PHARMACEUTICAL CO., LTD.
Tokyo 102-8667 (JP)**

(72) Inventors:
- **HASHIMOTO, Masaya
BANYU PHARMACEUTICAL CO., LTD.
Tsukuba-shi Ibaraki 300-2611 (JP)**
- **IWASAWA, Yoshikazu
BANYU PHARMACEUTICAL CO., LTD.
Tsukuba-shi, Ibaraki 300-2611 (JP)**
- **KAWAMOTO, Hiroshi
BANYU PHARMACEUTICAL CO., LTD.
Tsukuba-shi, Ibaraki 300-2611 (JP)**
- **OHTA, Hisashi
BANYU PHARMACEUTICAL CO., LTD.
Tsukuba-shi, Ibaraki 300-2611 (JP)**
- **OZAKI, Satoshi
BANYU PHARMACEUTICAL CO., LTD.
Tsukuba-shi, Ibaraki 300-2611 (JP)**
- **SAGARA, Takeshi
BANYU PHARMACEUTICAL CO., LTD.
Tsukuba-shi, Ibaraki 300-2611 (JP)**
- **SAKOH, Hiroki
BANYU PHARMACEUTICAL CO., LTD.
Tsukuba-shi, Ibaraki 300-2611 (JP)**
- **SATOH, Atsushi
BANYU PHARMACEUTICAL CO., LTD.
Tsukuba-shi, Ibaraki 300-2611 (JP)**

(74) Representative: **Hussain, Deeba et al
Merck & Co., Inc.
Hertford Road
Hoddesdon
Hertfordshire EN11 9BU (GB)**

(56) References cited:
**EP-A- 0 990 653**
**WO-A-01/39775**
**WO-A1-96/13262**
**WO-A1-97/40035**
**WO-A1-98/54168**
**JP-A- 48 000 474**
**JP-A- 2002 509 148**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **KAWAMOTO H. ET AL.: 'Discovery of the first potent and selective small molecule opioid receptor-like (ORL1) antagonist: 1-[(3R,4R)-1-cyclooctylmethyl-3-hydroxymeth yl-4-piperidyl]-3-ethyl-1,3-dihydro-2H-benz imidazol-2-one (J-113397)' J. MED. CHEM. vol. 42, no. 25, 1999, pages 5061 - 5063, XP003002987**

## Description

### Technical Field

This invention relates to substances which exhibit an antagonism to binding of nociceptin to nociceptin receptor ORL1 (Opioid receptor-like-1 receptor).

Compounds which inhibit binding of nociceptin to nociceptin receptor ORL1 are useful as analgesic against diseases accompanied with pain such as cancerous pain, postoperative pain, migraine, gout, chronic rheumatism, chronic pain and neuralgia; relievers against tolerance to narcotic analgesic represented by morphine; relievers against dependence on narcotic analgesic represented by morphine or against addiction; analgesic enhancers; antiobestic or appetite suppressors; treating or prophylactic agents for cognitive impairment and dementia/ amnesia in aging, cerebrovascular diseases and Alzheimer's disease; agents for treating developmental cognitive abnormality in attention deficit, hyperactivity disorder and learning disability; remedy for schizophrenia; agents for treating neurodegenerative diseases represented by Parkinsonism and chorea; anti-depressant or treating agents for affective disorder; treating or prophylactic agents for diabetes insipidus; treating or prophylactic agents for polyuria; and remedy for hypotension and the like.

### Background Art

Nociceptin (the same substance as orphanin FQ) is a peptide consisting of 17 amino acid units having a similar structure to that of opioid peptide. Nociceptin has an activity on reactivity against nociceptive stimulation, appetite stimulating activity, activity for reducing space learning ability, antagonism against analgesic action of classic opiate agonists, dopamine release inhibitory action, water diuresis action, vasodilative action and systemic blood pressure-lowering action, and it is considered to take part in intracerebral controlling of pain, appetite and memory learning through a nociceptin receptor ORL1 [cf. Nature, 377, 532 (1995); Society for Neuroscience, 22, 455 (1996); NeuroReport, 8, 423 (1997); Eur. J. Neuroscience, 9, 194 (1997); Neuroscience, 75, 1 (1996); ibid., 333(1996); Life Sciences, 60, PL15 (1997); ibid., PL141(1997); Proceedings for National Academy of Sciences, 94,14858 (1997)].

Further, it is known that morphine tolerance is reduced or memory and learning ability are improved in knockout mice in which expression of nociceptin receptor ORL1 is inhibited [cf. Neuroscience Letters, 237, 136 (1997); Nature, 394, 577 (1998)].

It has also been reported that nociceptin itself induces symptoms resembling withdrawal symptoms observed with morphine addicts, and that non-peptide nociceptin receptor antagonist improves morphine tolerance, dependence and symptoms resembling withdrawal symptoms [cf. Psychopharmacology, 151, 344-350 (2000); Journal of Neuroscience, 20, 7640 (2000)].

On the other hand, nociceptin protein precursor-defective mice are reported to show behaviors resembling anxiety and changes in stress response [cf. Proceedings for National Academy of Sciences, 96, 10444 (1999)].

Hence the substances which specifically inhibit binding of nociceptin to nociceptin receptor ORL1 are useful as analgesic against diseases accompanied with pain such as cancerous pain, postoperative pain, migraine, gout, chronic rheumatism, chronic pain and neuralgia; relievers against tolerance to narcotic analgesic represented by morphine; relievers against dependence on narcotic analgesic represented by morphine or against addiction; analgesic enhancers; antiobestic or appetite suppressors; treating or prophylactic agents for cognitive impairment and dementia/ amnesia in aging, cerebrovascular diseases and Alzheimer's disease; agents for treating developmental cognitive abnormality in attention deficit, hyperactivity disorder and learning disability; remedy for schizophrenia; agents for treating neurodegenerative diseases represented by Parkinsonism and chorea; anti-depressant or treating agents for affective disorder; treating or prophylactic agents for diabetes insipidus; treating or prophylactic agents for polyuria; and remedy for hypotension and the like.

International Publication WO98/54168 or J. Med Chem. 5061-5063(1999) disclose compounds having antagonism to binding of nociceptin to nociceptin receptor ORLI. In particular, the compound of the following formula (A)

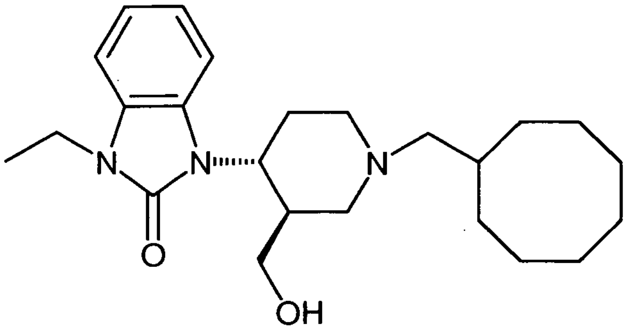

[A]

(hereinafter referred to as "Compound A") is disclosed as having excellent selective antagonism to binding of nociceptin to nociceptin receptor.
International Publication WO98/54168.
J. Med. Chem., 1999, 5061-5063

Further nociceptin receptor ORL1 antagonists are disclosed in WO 01/39775.

Disclosure of the Invention

We have investigated on compounds of analogous structures to that of Compound A in search for compounds which exhibit antagonistic activity to binding of nociceptin to nociceptin receptor ORL1, to discover that those compounds having bi-or tri-cyclic aliphatic carbocyclic group of specific carbon numbers in place of the cyclooctyl group and also having dihydroxyalkyl substituent group on the nitrogen atom possess well balanced activities of not only selectively inhibiting binding of nociceptin to nociceptin receptor but also exhibiting excellent in vivo metabolic properties, and can be the compounds particularly suitable for application to human being. The present invention is completed based on that discovery.

Thus, the present invention provides

(1) 2-oxoimidazole derivatives represented by the formula (I)

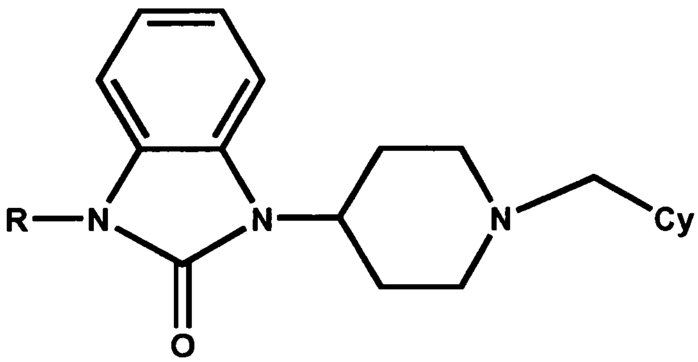

(I)

in which, R stands for a di-hydroxy-substituted $C_1$ - $C_6$ alkyl group, and Cy is a spiro[4.5]dec-6-yl, spiro[2.5]oct-4-yl, spiro[3.5]non-5-yl, 3,3-dimethylbicyclo[2.2.1]hept-2-yl or 1-spiro(bicyclo[2.2.1]heptane-2,1'cyclopropan)-3-yl group, or a pharmaceutically acceptable salt thereof

The invention furthermore provides

(2) A pharmaceutical composition comprising a pharmaceutically acceptable adjuvant and a compound as described in (1) above or a pharmaceutically acceptable salt thereof; and
(3) A method for the manufacture of a medicament for use as an analgesic; reliever against tolerance to narcotic analgesics; reliever against dependence on narcotic analgesics or against addiction; analgesic enhancer; antiobestic or appetite suppressor; agent for cognitive impairment and dementia/amnesia in aging, cerebrovascular disease

and Alzheimer's disease; agent for treating developmental cognitive abnormality in attention deficit, hyperactivity disorder and learning disability; remedy for schizophrenia; agent for treating neurodegenerative diseases represented by Parkinsonism and chorea; anti-depressant or treating agents for affective disorder; treating or prophylactic agent for diabetes insipidus; treating or prophylactic agent for polyuria; and remedy for hypotension; which comprises combining a compound described in (1) above or a pharmaceutically acceptable salt thereof, and a pharmaceutically acceptable adjuvant.

BEST MODE FOR CARRYING OUT THE INVENTION

Hereinafter the invention is explained in details, referring to specific examples.

In the formula (I), R stands for a $C_1$ - $C_6$ alkyl group having two hydroxyl groups, specific examples including 2-hydroxy-1-(hydroxymethyl)ethyl, 2,3-dihydroxypropyl, 2,3-dihydroxy-2-methylpropyl, 2,3-dihydroxybutyl, 2,4-dihydroxybutyl, 3,4-dihydroxybutyl, 2,3-dihydroxy-1-methylpropyl, 2-hydroxy-1-(hydroxymethyl)propyl, 3-hydroxy-1-(hydroxymethyl)propyl, 3-hydroxy-2-(hydroxymethyl)propyl, 2,3-dihydroxypentyl, 2,4-dihydroxypentyl, 2,5-dihydroxypentyl, 3,4-dihydroxypentyl, 3,5-dihydroxypentyl, 4,5-dihydroxypentyl, 2,3-dihydroxy-1-methylbutyl, 2,4-dihydroxy-1-methylbutyl, 3,4-dihydroxy-1-methylbutyl, 2-hydroxy-1-(hydroxymethyl)butyl, 3-hydroxy-1-(hydroxymethyl)butyl, 4-hydroxy-1-(hydroxymethyl)butyl, 2,3-dihydroxy-2-methylbutyl, 2,4-dihydroxy-2-methylbutyl, 3,4-dihydroxy-2-methylbutyl, 2-hydroxy-2-(hydroxymethyl)butyl, 3-hydroxy-2-(hydroxymethyl)butyl, 4-hydroxy-2-(hydroxymethyl)butyl, 2,3-dihydroxy-3-methylbutyl, 2,4-dihydroxy-3-methylbutyl, 3,4-dihydroxy-3-methylbutyl, 4-hydroxy-3-(hydroxymethyl)butyl, 2,3-dihydroxy-1,1-dimethylpropyl,2-hydroxy-1-(hydroxymethyl)-1-methylpropyl, 3-hydroxy-1-(hydroxymethyl)-1-methylpropyl, 1,1-bis(hydroxymethyl)propyl, 2,3-dihydroxy-1,2-dimethylpropyl, 2-hydroxy-1-(hydroxymethyl)-2-methylpropyl, 3-hydroxy-2-(hydroxymethyl)-2-methylpropyl, 2,3-dihydroxy-1-ethylpropyl, 2-hydroxy-1-(2-hydroxyethyl)propyl, 2-hydroxy-1-(1-hydroxyethyl)propyl, 3-hydroxy-1-(2-hydroxyethyl)propyl, 2,3-dihydroxyhexyl, 2,4-dihydroxyhexyl, 2,5-dihydroxyhexyl, 2,6-dihydroxyhexyl, 3,4-dihydroxyhexyl, 3,5-dihydroxyhexyl, 3,6-dihydroxyhexyl, 4,5-dihydroxyhexyl and 4,6-dihydroxyhexyl. Preferred examples are $C_3$ - $C_4$ alkyl groups having two hydroxyl group and, in particular, 2,3-dihydroxypropyl, 2-hydroxy-1-(hydroxymethyl)ethyl and 2,3-dihydroxy-2-methylpropyl groups are recommended.

Cy stands for spiro[4.5]dec-6-yl, spiro[2.5]oct-4-yl, spiro[3.5]non-5-yl, 3,3-dimethylbicyclo[2.2.1]hept-2-yl, or 1-spiro(bicyclo[2.2.1]heptane-2,1'-cyclopropan)-3-yl.

According to the invention, by the adoption of those 2-oxoimidazole derivatives of formula (I), compounds having very well balanced physiological activity of excellent antagonism to nociceptin receptor and also excellent metabolic stability can be provided.

As specific examples of the compounds represented by the formula (I), the following can be named:

1) 1-(2,3-dihydroxypropyl)-3-[1-spiro[4.5]-dec-6-ylmethyl]-piperidin-4-yl]-1,3-dihydro-2H-benzimidazol-2-one,
2) 1-[(2R)-2,3-dihydroxypropyl]-3- {1-[(6S)-spiro[4.5]dec-6-ylmethyl]piperidin-4-yl}-1,3-dihydro-2H-benzimidazol-2-one,
3) 1-[(2R)-2,3-dihydroxypropyl]-3-{1-[(6R)-spiro[4.5]dec-6-ylmethyl]piperidin-4-yl}-1,3-dihydro-2H-benzimidazol-2-one,
4) 1-[(2S)-2,3-dihydroxypropyl]-3-{1-[(6S)-spiro[4.5]dec-6-ylmethyl]piperidin-4-yl}-1,3-dihydro-2H-benzimidazol-2-one,
5) 1-[(2S)-2,3-dihydroxypropyl]-3-{1-[(6R)-spiro[4.5]dec-6-yhnethyl]piperidin-4-yl}-1,3-dihydro-2H-benzimidazol-2-one,
6) 1-(2,3-dihydroxypropyl)-3-[1-(spiro-[3.5]non-5-ylmethyl)-piperidin-4-yl]-1,3-dihydro-2H-benzimidazol-2-one,
7) 1-[(2R)-2,3-dihydroxypropyl]-3-{1-[(5S)-spiro[3.5]non-5-ylmethyl]piperidin-4-yl}-1,3-dihydro-2H-benzmidazol-2-one,
8) 1-[(2R)-2,3-dihydroxypropyl]-3-{1-[(5R)-spiro[3.5]non-5-ylmethyl]piperidin-4-yl}-1,3-dihydro-2H-benzimidazol-2-one,
9) 1-[(2S)-2,3-dihydroxypropyl]-3-{1-[(5S)spiro[3.5]non-5-ylmethyl]piperidin-4-yl}-1,3-dihydro-2H-benzimidazol-2-one,
10) 1-[(2S)-2,3-dihydroxypropyl]-3-{1-[(5R)-spiro[3.5]non-5-ylmethyl]piperidin-4-yl}-1,3-dihydro-2H-benzimidazol-2-one,
11) 1-(2,3-dihydroxypropyl)-3-{1-[(3,3-dimethylbicyclo[2.2.1]-hept-2-yl)methyl]piperidin-4-yl}-1,3-dihydro-2H-benzimidazol-2-one,
12) 1-[(2R)-2,3-dihydroxypropyl]-3-(1-{[(1S, 2S, 4R)-3,3-dimethylbicyclo[2.2.]hept-2-yl]methyl}piperidin-4-yl)-1,3-dihydro-2H-benzimidazol-2-one,
13) 1-[(2R)-2,3-dihydroxypropyl]-3-(1-{[(1S, 2R, 4R)-3,3-dimethylbicyclo[2.2.1]hept-2-yl]methyl}piperidin-4-yl)-1,3-dihydro-2H-benzimidazol-2-one,
14) 1-[(2R)-2,3-dihydroxypropyl]-3-(1-{[(1R,2R, 4S)-3,3-dimethylbicyclo[2.2.1]hept-2-yl]methyl}piperidin-4-yl)1,3-dihydro-2H-benzimidazol-2-one,
15) 1-[(2R)-2,3-dihydroxypropyl]-3-(1-{[(1R, 2S, 4S)-3,3-dimethylbicyclo[2.2.1]hept-2-yl]methyl}piperidin-4-yl)-1,3-dihydro-2H-benzimidazol-2-one,
16) 1-[(2S)-2,3-dihydroxypropyl]-3-(1-{[(1S,2S,4R)-3,3-dimethylbicyclo[2.2.1]hept-2-yl]methyl}piperidin-4-yl)-1,3-dihydro-2H-benzimidazol-2-one,
17) 1-[(2S)-2,3-dihydroxypropyl]-3-(1-{[(1S, 2R, 4R)-3,3-dimethylbicyclo[2.2.1]hept-2-yl]methyl}piperidin-4-yl)-1,3-dihydro-2H-benzimidazol-2-one,
18) 1-[(2S)-2,3-dihydroxypropyl]-3-(1-{[(1R, 2R, 4S)-3,3-dimethylbicyclo[2.2.1]hept-2-yl]methyl}piperidin-4-yl)-1,3-dihydro-2H-benzimidazol-2-one,
19) 1-[(2S)-2,3-dihydroxypropyl]-3-(1-{[(1R, 2S, 4S)-3,3-dimethylbicyclo[2.2.1]hept-2-yl]methyl}piperidin-4-yl)-1,3-dihydro-2H-benzimidazol-2-one,
20) 1-(2,3-dihydroxypropyl)-3-[1-(spiro[2.5]oct-4-ylmethyl)-piperidin-4-yl]-1,3-dihydro-2H-benzimidazol-2-one,
21) 1-[(2R)-2,3-dihydroxypropyl]-3-{1-[(4S)-spiro[2.5]oct-4-ylmethyl]piperidin-4-yl}-1,3-dihydro-2H-benzimidazol-2-one,
22) 1-[(2R)-2,3-dihydroxypropyl]-3-{1-[(4R)-spiro[2.5]oct-4-ylmethyl]piperidin-4-yl}-1,3-dihydro-2H-benzimidazol-2-one,
23) 1-[(2S)-2,3-dihydroxypropyl]-3-{1-[(4S)-spiro[2.5]oct-4-ylmethyl]piperidin-4-yl}-1,3-dihydro-2H-benzimidazol-2-one,
24) 1-[(2S)-2,3-dihydroxypropyl]-3-{1-[(4R)-spiro[2.5]oct-4-ylmethyl]piperidin-4-yl}-1,3-dihydro-2H-benzimidazol-2-one,
25) 1-(2,3-dihydroxypropyl)-3-[1-(spiro[bicyclo[2.2.1]heptane-2,1'-cyclopropan]-3-ylmethyl)piperidin-4-yl]-1,3-dihydro-2H-benzimidazol-2-one,
26) 1-[(2R)-2,3-dihydroxypropyl]-3-{1-[(1R, 3S, 4S)-spiro[bicyclo[2.2.1]heptane-2,1'-cyclopropan]-3-ylmethyl]piperidin-4-yl}-1,3-dihydro-2H-benzimidazol-2-one,
27) 1-[(2R)-2,3-dihydroxypropyl]-3-{1-[(1R, 3R, 4S)-spiro-[bicyclo[2.2.]heptane-2,1'-cyclopropan]-3-ylmethyl]piperidin-4-yl}-1,3-dihydro-2H-benzimidazol-2-one,
28) 1-[(2R)-2,3-dihydroxypropyl]-3-{1-[(1S, 3R, 4R)-spiro-[bicyclo[2.2.1]heptane-2,1'-cyclopropan]-3-ylmethyl]piperidin-4-yl} 1,3-dihydro-2H-benzimidazol-2-one,
29) 1-[(2R)-2,3-dihydroxypropyl]-3-{1-[(1S, 3S, 4R)-spiro-bicyclo[2.2.1]heptane-2,1'-cyclopropan]-3-ylmethyl]piperidin-4-yl}-1,3-dihydro-2H-benzimidazol-2-one,
30) 1-[(2S)-2,3-dihydroxypropyl]-3-{1-[(1R, 3S, 4S)-spiro-[bicyclo[2.2.1]heptane-2,1'-cyclopropane]-3-ylmethyl]piperidin-4-yl}-1,3-dihydro-2H-benzimidazol-2-one,
31) 1-[(2S)-2,3-dihydroxypropyl]-3-{1-[(1R, 3R, 4S)-spiro-[bicyclo[2.2.]heptane-2,1'-cyclopropan]-3-ylmethyl]piperidin-4-yl}-1,3-dihydro-2H-benzimidazol-2-one,
32) 1-[(2S)-2,3-dihydroxypropyl]-3-{1-[(1S, 3R, 4R)-spiro-[bicyclo[2.2.]heptane-2,1'-cyclopropan]-3-ylmethyl]piperidin-4-yl}-1,3-dihydro-2H-benzimidazol-2-one,
33) 1-[(2S)-2,3-dihydroxypropyl]-3-{1-[(1S, 3S, 4R)-spiro-[bicyclo[2.2.]heptane-2,1'-cyclopropan]-3-ylmethyl]piperidin-4-yl}-1,3-dihydro-2H-benzimidazol-2-one,
34) 1-[2-hydroxy-1-(hydroxymethyl)ethyl]-3-[1-(spiro[2.5]-oct-4-ylmethyl)piperidin-4-yl]-1,3-dihydro-2H-benzimidazol-2-one,
35) 1-[2-hydroxy-1-(hydroxymethyl)ethyl]-3-{1-[(4R)-spiro-[2.5]oct-4-ylmethyl]piperidin-4-yl}-1,3-dihydro-2H-benzimidazol-2-one,
36) 1-[2-hydroxy-1-(hydroxymethyl)ethyl]-3-{1-[(4S)-spiro[2.5]-oct-4-ylmethyl]piperidin-4-yl}-1,3-dihydro-2H-benzimidazol-2-one,
37) 1-[2-hydroxy-1-(hydroxymethyl)ethyl-3-[1-(spiro[bicyclo-[2.2.1]heptane-2,1'-cyclopropan]-3-ylmethyl)piperidin-4-yl]-1,3-dihydro-2H-benzimidazol-2-one,
38) 1-[2-hydroxy-1-(hydroxymethyl)ethyl]-3-{1-[(1R, 3S, 4S)-spiro[bicyclo[2.2.1]heptane-2,1'-cyclopropan]-3-ylmethyl]piperidin-4-yl}-1,3-dihydro-2H-benzimidazol-2-one,
39) 1-[2-hydroxy-1-(hydroxymethyl)ethyl]-3-{1-[(1R, 3R, 4S)-spiro[bicyclo[2.2.1]heptane-2,1'-cyclopropan]-3-ylmethyl]piperidin-4-yl}-1,3-dihydro-2H-benzimidazol-2-one,
40) 1-[2-hydroxy-1-(hydroxymethyl)ethyl]-3-{1-[(1S, 3R, 4R)-spiro[bicyclo[2.2.1]heptane-2,1'-cyclopropan]-3-ylmethyl]piperidin-4-yl}-1,3-dihydro-2H-benzimidazol-2-one,
41) 1-[2-hydroxy-1-(hydroxymethyl)ethyl]-3-{1-[(1S, 3S, 4R)-spiro[bicyclo[2.2.1]heptane-2,1'-cyclopropan]-3-ylmethyl]piperidin-4-yl}-1,3-dihydro-2H-benzimidazol-2-one,
42) 1-(2,3-dihydroxy-2-methylpropyl)-3-[1-(spiro[2.5]oct-4-ylmethyl)piperidin-4-yl]-1,3-dihydro-2H-benzimidazol-2-one,
43) 1-[(2R)-2,3-dihydroxy-2-methylpropyl]-3-{1-[(4S)-spiro[2.5]-oct-4-ylmethyl]piperidin-4-yl}-1,3-dihydro-2H-benzimidazol-2-one,

44) 1-[(2R)-2,3-dihydroxy-2-methylpropyl]-3-{1-[(4R)-spiro[2.5]-oct-4-ylmethyl]piperidin-4-yl}-1,3-dihydro-2H-benzimidazol-2-one,
45) 1-[(2S)-2,3-dihydroxy-2-methylpropyl]-3-{1-[(4S)-spiro[2.5]-oct-4-ylmethyl]piperidin-4-yl}-1,3-dihydro-2H-benzimidazol-2-one, and
46) 1-[(2S)-2,3-dihydroxy-2-methylpropyl]-3-{1-[(4R)-spiro[2.5]-oct-4-ylmethyl]piperidin-4-yl}-1,3-dihydro-2H-benzimidazol-2-one.

Preferably,

1-[(2R)-2,3-dihydroxypropyl]-3-{1-(1R, 3S, 4S)-spiro[bicyclo-[2.2.1]heptane-2,1'-cyclopropan]-3-ylmethyl]piperidin-4-yl}-1,3-dihydro-2H-benzimidazol-2-one,

1-[(2S or 2R)-2,3-dihydroxy-2-methylpropyl-3- {1-[(4S)-spiro[2.5]oct-4-ylmethyl]piperidin-4-yl}-1,3-dihydro-2H-benzimidazol-2-one,

1-[2-hydroxy-1-(hydroxymethyl)ethyl]-3-{1-[(1R, 3S, 4S)-spiro-[bicyclo[2.2.]heptane-2,1'-cyclopropan]-3-ylmethyl] piperidin-4-yl}-1,3-dihydro-2H-benzimidazol-2-one,

1-[(2R)-2,3-dihydroxypropyl]-3-{1-[(4S)-spiro[2.5]oct-4-ylmethyl]piperidin-4-yl}-1,3-dihydro-2H-benzimidazol-2-one and

1-[(2R)-2,3-dihydroxypropyl]-3-[1-[(6S or 6R)-spiro[4.5]dec-6-ylmethyl]piperidin-4-yl}-1,3-dihydro-2H-benzimidazol-2-one are recommended.

<u>Production processes of the compounds represented by the formula (I)</u>

Those compounds represented by the formula (I) can be prepared by following production processes or also by the processes as described in WO98/54168.

Production process 1

Production process 1 uses 1,3-dihydro-1-(4-piperidinyl)-2H-benzimidazol-2-one which is a known compound, and provides compounds of the formula (I) through three- or four- stage steps.

reaction scheme 1

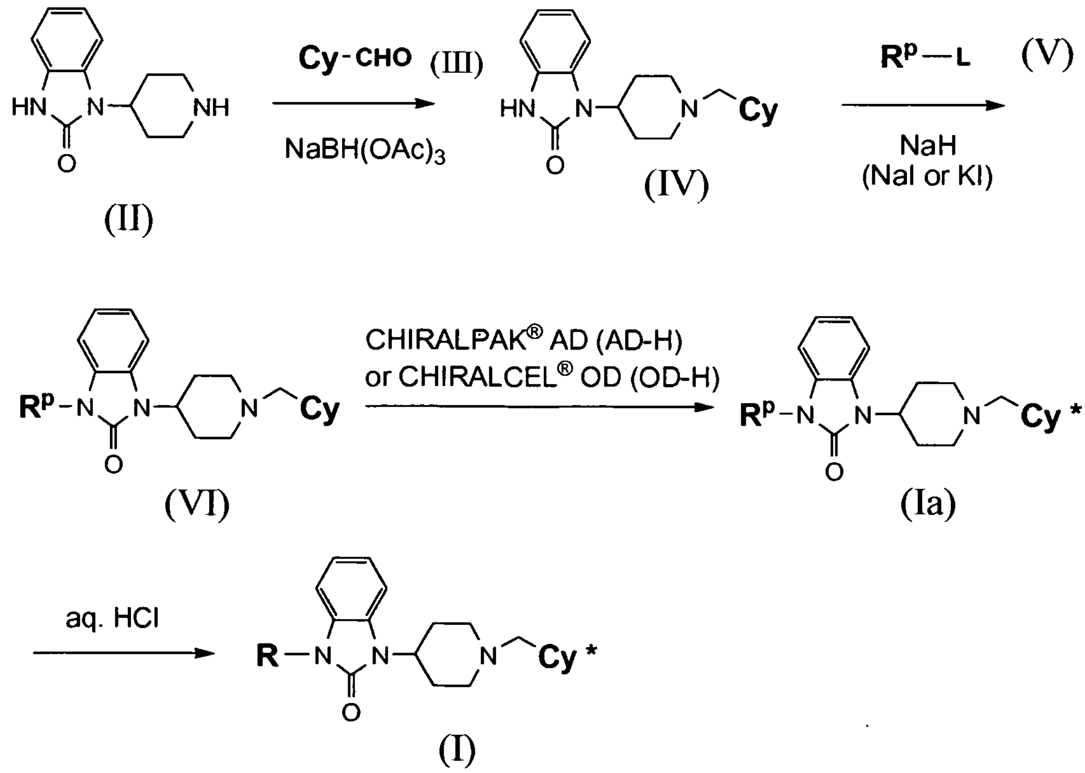


in which, $R^P$ stands for a lower alkyl group having two protected hydroxyl groups, L stands for a leaving group, Cy* stands for an optically active Cy, and Cy and R have the same significations as defined earlier.

A compound of the formula (II) and a compound of the formula (III) are subjected to a reductive alkylation reaction in an organic solvent in the presence of a reducing agent, to provide a compound of the formula (IV).

As the use rate of the compounds of the formulae (II) and (III), respectively, they are normally used in equimolar amounts, or either one of them is used in slight molar excess.

As the reducing agent, for example, sodium cyanoborohydride, sodium triacetoxyborohydride, zinc biscyanoborohydride, nickel biscyanoborohydride and the like can be named.

As the use rate of the reducing agent, it may be a mol to molar excess, preferably 1 - 5 mols, per mol of the compound represented by the formula (II).

The reaction is normally carried out in organic solvent. Examples of useful solvent include alcohols such as methanol, ethanol and propanol; ethers such as diethyl ether, tetrahydrofuran ("THF") and dioxane; halogenated hydrocarbons such as methylene chloride, chloroform and dichloroethane; aromatic hydrocarbons such as benzene, toluene, chlorobenzene and xylene; and aprotic polar solvents such as dimethylformamide ("DMF"), acetonitrile and hexamethylphosphorictriamide, or mixed solvents of the foregoing.

Exemplary reaction temperature normally ranges -20°C - 100°C, preferably 0°C - room temperature, and the reaction time ranges normally from 5 minutes to 7 days, preferably 1 - 6 hours.

As the compounds represented by the formula (III), for example, the following compounds can be used.

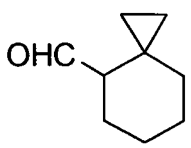 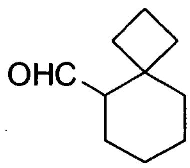 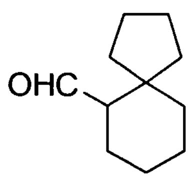 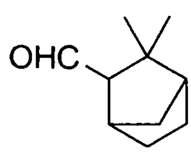 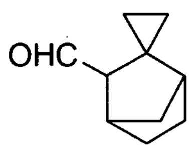

Then the compound of the formula (IV) is condensed with a compound of the formula (V) in an organic solvent

in the presence of a base, to provide a compound of the formula (VI).

As the use rate of the compound of the formula (V), it may range from a mol to molar excess, preferably 1 - 5 mols, per mol of the compound of the formula (IV).

Examples of useful base include sodium hydride, potassium hydride, lithium hexamethyldisilazide, sodium hexamethyldisilazide, potassium hexamethyldisilazide, potassium carbonate and sodium carbonate. Preferably, sodium hydride and potassium hydride are recommended.

As the use rate of such a base, for example, it may range from a mol to molar excess, preferably 1 - 5 mols, per mol of the compound of the formula (V).

An alkali metal halide such as sodium iodide, potassium iodide or the like may be added to the reaction system for promoting the reaction. As the use rate in such an occasion, for example, 0.1 mol - molar excess of an alkali metal halide per mol of the compound of the formula (IV) can be used.

As the organic solvent, for example, DMF, THF, hexamethylphosporictriamide and the like can be named.

Exemplary reaction temperature normally ranges 0°C - 150°C, preferably room temperature -130°C being recommended. The reaction time normally ranges 5 minutes to 7 days, preferably an hour - 12 hours.

In the compound of the formula (V), L stands for a leaving group which can be, for example, benzenesulfonyloxy, p-toluenesulfonyloxy, methanesulfonyloxy, fluorine, chlorine or bromine.

Specific examples of the compounds represented by the formula (V) include the following.

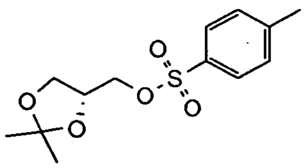
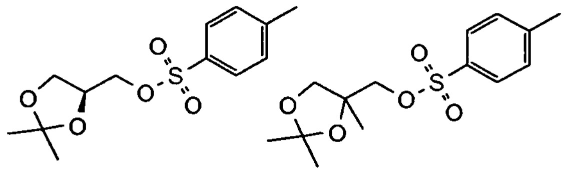
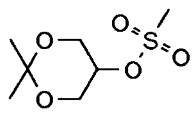

Successively the compound of the formula (VI) is optically resolved with optically active column, where necessary, to provide an optically active compound of the formula (Ia), and then the protective groups of the hydroxyl groups in the compound of the formula (Ia) are removed to provide a compound of the formula (I).

Such hydroxyl-protective groups are subject to no particular limitation, so long as they have the required function. For example, such groups as tert-butyl; alkylsilyl, e.g., trimethylsilyl, tert-butyldimethylsilyl and tert-butyldiphenylsilyl; methoxymethyl; tetrahydropyranyl; trimethylsilylethoxymethyl; aralkyl, e.g., benzyl, p-methoxybenzyl, 2,3-dimethoxybenzyl, o-nitrobenzyl, p-nitrobenzyl and trityl; and acyl, e.g., formyl and acetyl can be named, among which methoxymethyl, tetrahydropyranyl, trityl, trimethylsilylethoxymethyl, tert-butyldimethylsilyl and acetyl are particularly preferred.

In particular, as protective groups of 1,2- or 1,3-diols, for example, methyleneketal, ethylideneacetal, phenylethylideneacetal, 4-methoxyphenylethylideneacetal, isopropylideneketal and benzylideneacetal can be named.

Means for removing protective groups differ depending on kind of protective groups and stability of individual compounds represented by formula [Ia]. For example, the removal is conducted following those methods describe in literature [cf. Protective Groups in Organic Synthesis, T. W. Greene, John Wiley & Sons Co., (1981)] or those analogous thereto, by solvolysis using acid or base, i.e., a method of having, for example, from 0.01 mol to a large molar excess of acid, preferably trifulroacetic acid, formic acid, hydrochloric acid or the like; or from equimolar to a large molar excess of base, preferably potassium hydroxide, calcium hydroxide or the like, act on the object compound; chemical reduction using hydrogenated metal complex or by catalytic reduction using palladium-on-carbon catalyst or Raney nickel catalyst.

In case of diol-protective groups such as ketal, acetal and the like, the deprotection can be effected by hydrolyzing the compound of the formula (VI) using hydrochloric acid, in a solvent such as THF, dioxane or the like, at room temperature -100°C.

Where necessary, the compound of the formula (VI) can be optically resolved by means of chromatography using optically active column, to provide an optically active compound of the formula (Ia).

As the optically active column, for example CHIRALPAK® AD, CHIRALPAK® AD-H, CHIRAL CELL® OD and CHIRAL CELL® OD-H (Daicel Co., Ltd.) can be named.

As the eluent solvent in that occasion, mixed solvents such as hexane/2-propanol/diethylamine = 1900/100/2 - 800/200/1 by volume, or hexane/ethanol/diethylamine = 1900/100/2 - 800/200/1 by volume can be used.

As the detection means of the compounds in such occasion, for example, ultraviolet rays in the wavelength region near 280 nm may be used.

Production process 2

Production process 2 is one for making compounds of the formula (I), using the formula (IV) compounds as the starting material.

reaction scheme 2

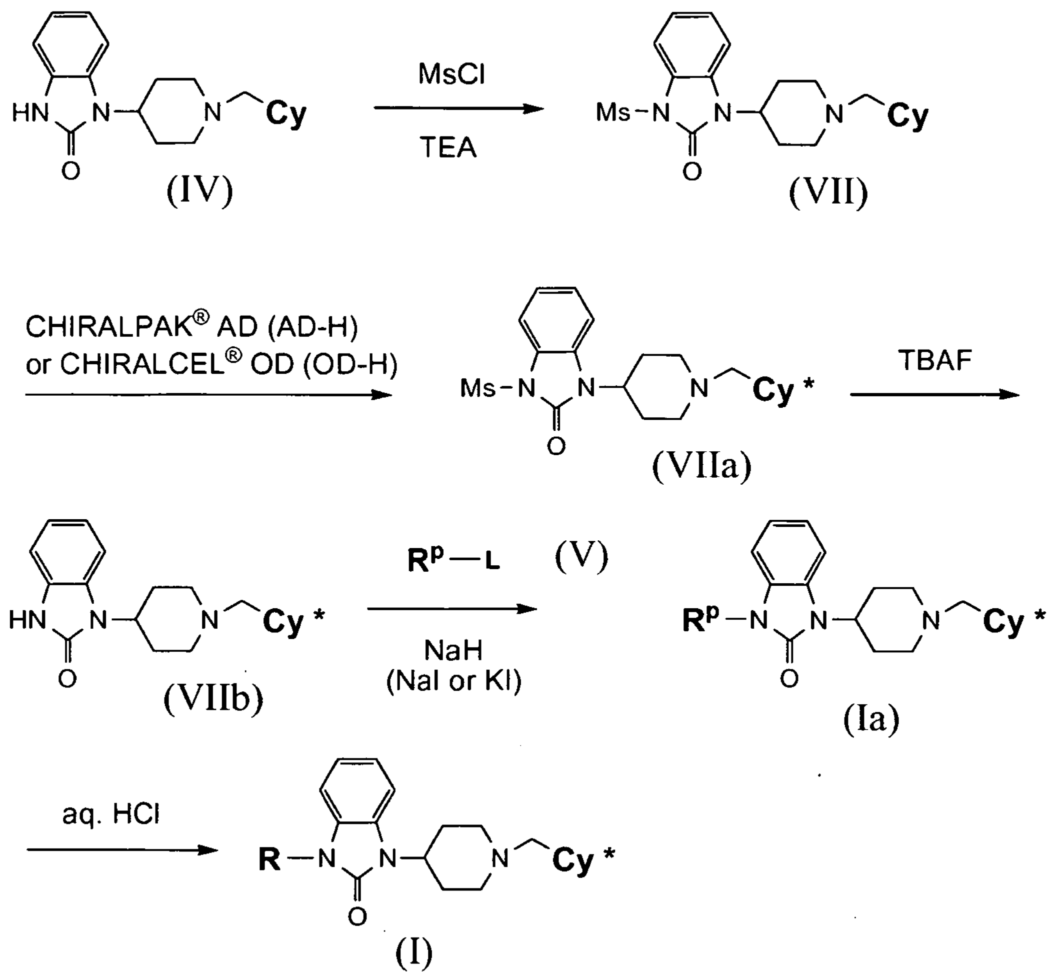


in which, Ms stands for methanesulfonyl group, TEA stands for triethylamine, and Cy, Cy*, $R^p$, L and R have the same significations as previously defined.

A compound of the formula (IV) is mesylated by a means known <u>per se</u>, to be converted to a compound of the formula (VII) which is successively optically resolved according to the production process 1 to provide a compound of the formula (VIIa). Further, mesyl group in a compound of the formula (VIIa) is removed using tetra-n-butylammonium fluoride (TBAF) to provide a compound of the formula (VIIb) which is reacted with the compound of the formula (V) according to the production process 1 to provide the compound of the formula (Ia) which is further converted to the compound of the formula (I) by deprotection.

So obtained compound of the formula (I) can be easily isolated and purified by ordinary separation means, for example, solvent extraction, recrystallization, column chromatography, preparative thin layer chromatography or the like.

These compounds can be converted to pharmaceutically acceptable salts according to accepted practice. Conversely, conversion from salts to free compounds can also be conducted by conventionally practiced means.

As examples of salts of compounds of the formula (I), acid addition salts at the piperidinyl group can be named.

As examples of such acid addition salts, inorganic acid salts such as hydrochloride, sulfate, nitrate, phosphate, perchlorate and the like; carboxylic acid salts such as maleate, fumarate, tartrate, citrate, ascorbate, trifuoroacetate and the like; and sulfonates such as methanesulfonate, isethionate, benzenesulfonate, p-toluenesulfonate and the like can be named.

Action of compounds of the present invention as nociceptin receptor antagonist is shown, for example, by the following pharmacological test examples.

Pharmacological Test Example 1 (nociceptin receptor binding inhibition assay)

cDNA which codes a human nociceptin receptor gene was integrated with an expression vector pCR3 (Invitrogen) to prepare pCR3 / ORL1. Next, pCR3 / ORL1 was transfected in CHO cells using a transfectam (Nippongene) to obtain a stable expression strain (CHO / ORL1 cells) having resistance against 1 mg / ml G418. Membrane fractions were prepared from this stable expression strain to carry out a receptor binding assay. The membrane of 11 μg, 50 pM [$^{125}$I] Tyr$^{14}$-Nociceptin (Amersham Pharmacia), 1 mg Wheatgerm agglutinin SPA beads (PVT based; Amersham Pharmacia) and each test compound were suspended in an NC buffer (50 mM Hepes, 10 mM sodium chloride, 1 mM magnesium chloride, 2.5 mM calcium chloride, 0.1% BSA, 0.025% bacitracin, pH 7.4) and incubated at 37°C for 60 minutes, and then the radioactivity was determined. The binding activity to the nociceptin receptor was indicated by the 50% inhibition concentration ($IC_{50}$ value) of [$^{125}$I] Tyr$^{14}$-Nociceptin binding of each test compound. The results were as shown in Table 1.

TABLE 1

| Compound | $IC_{50}$ value (nM) |
|---|---|
| Example 1 | 1.6 |
| Example 2 | 5.1 |
| Example 3 | 8.7 |
| Example 4 | 1.9 |
| Example 5 | 2.8 |

Pharmacological Test Example 2 (antagonism against nociceptin-elicited G protein activation)

CHO cells which stably represented nociceptin receptor ORL1 were used to investigate the action of each test compound against nociceptin-elicited G protein activation. A membrane prepared from the CHO / ORL1 cells, 50 nM nociceptin, 200 pM GTPγ[$^{35}$S] (NEN), 1.5mg Wheatgerm agglutinin SPA beads (Amersham Pharmacia) and each of the test compounds were mixed in a GDP buffer (20 mM Hepes, 100 mM sodium chloride, 10 mM magnesium chloride, 1 mM EDTA, 5 μM GDP, pH 7.4) and incubated at 25°C for 150 minutes, and then the radioactivity was determined. The antagonism against nociceptin-elicited G protein activation was shown by the 50% inhibition concentration ($IC_{50}$ value) of each test compound against GTPγ[$^{35}$S] binding. The results were as shown in Table 2.

TABLE 2

| Compound | 50% value (nM) |
|---|---:|
| Example 1 | 3.9 |
| Example 2 | 8.6 |
| Example 3 | 18.0 |
| Example 4 | 6.0 |
| Example 5 | 4.5 |

Pharmacological Test Example 3 (metabolic stability test)

Metabolic stability of test compounds was examined using human liver microsome. A 100mM potassium phosphate buffer (pH 7.4) comprising 10mM G-6-P, 1.0 mM $NADP^+$, 10 units/mL G-6-P DH, 3.0 mM $MgCl_2$ and 0.25 mg protein/mL of human liver microsome was prepared, each 392 μL of which was poured into plural vessels and preincubated at 37°C for 5 minutes. Then 8 μL each of 50 μM test compound (50% acetonitrile solution) was added to initiate the reaction (final test compound concentration: 1 μM). At the initiation time and 30 minutes thereafter of the reaction, 150 μL of each reaction solution was added to 450 μL of ethanol to suspend the reaction, followed by centrifugal separation (12,000 g, 12 minutes, 4°C). Resulting supernatant was analyzed with LC/MS/MS. Based on the peak area

of the test compound in each sample at the initiating time of the reaction as 100%, the test compound's residual ratio in the sample after 30 minutes' reaction was calculated. The results were as shown in Table 3.

$$\text{Residual ratio (\%)} = [\text{peak area (after 30 minutes' reaction)}/\text{peak area (0 minute's reaction)}] \times 100.$$

TABLE 3

| Compound | Residual Ratio (%) |
|---|---|
| Example 2 | 82 |
| Example 4 | 72 |
| Example 5 | 65 |

<u>Pharmaceutical preparations comprising compounds represented by the formula (I)</u>

The compounds of the present invention can be administered orally or parenterally and, as formulated into preparation forms suitable for such administration routes, can be used as analgesic against diseases accompanied by pain such as cancerous pain, postoperative pain, migraine, gout, chronic rheumatism, chronic pain and neuralgia; relievers against tolerance to narcotic analgesic represented by morphine; relievers against dependence on narcotic analgesic represented by morphine or against addiction; analgesic enhancer; antiobestic or appetite suppressors; treating or prophylactic agents for cognitive impairment and dementia/ amnesia in aging, cerebrovascular diseases and Alzheimer's disease; agents for treating developmental cognitive abnormality in attention deficit, hyperactivity disorder and learning disability; remedy for schizophrenia; agents for treating neurodegenerative diseases represented by Parkinsonism and chorea; anti-depressants or treating agents for affective disorder; treating or prophylactic agents for diabetes insipidus; treating or prophylactic agents for polyuria; remedy for hypotension, and the like.

In actually using the compounds of the present invention clinically, they can normally be formulated into various preparation forms suitable for individual mode of administration, with pharmaceutically acceptable adjuvants. As the adjuvants, various additives customarily used in the field of medical preparations can be used, examples of which including gelatin, lactose, sucrose, titanium oxide, starch, crystalline cellulose, hydroxypropylmethyl cellulose, carboxymethyl cellulose, corn starch, microcrystalline wax, white petrolatum, magnesium aluminate metasilicate, anhydrous calcium phosphate, citric acid, trisodium citrate, hydroxypropyl cellulose, sorbitol, sorbitan fatty acid ester, polysorbate, sucrose fatty acid ester, polyoxyethylene, hardened castor oil, polyvinylpyrrolidone, magnesium stearate, light silicic anhydride, talc, vegetable oil, benzyl alcohol, acacia, propylene glycol, polyalkylene glycol, cyclodextrin or hydroxypropyl cyclodextrin and the like.

As the forms of preparations formulated as pharmaceutical compositions using these adjuvants, solid preparations such as tablets, capsules, granules, powders and suppositories; liquid preparations such as syrups, elixirs and injections can be named. These preparations can be formulated according to conventional methods used in the field of pharmaceutics. Liquid preparations may be in a form which is dissolved or suspended in water or other suitable medium immediately prior to use. In particular, injections may be in the form of a solution or suspension in physiological saline solution or a glucose solution, to which a buffer agent, a preservative or the like may be added.

These preparations can contain a compound or compounds of the present invention at the ratios of 1 - 100 wt%, preferably 1 - 60 wt%, based on the total pharmaceutical preparation.
These preparations may further contain other therapeutically active compounds.

Where the compounds of the present invention are used as analgesic against diseases accompanied with pain such as cancerous pain, postoperative pain, migraine, gout, chronic rheumatism, chronic pain and neuralgia; relievers against tolerance to narcotic analgesic represented by morphine; relievers against dependence on narcotic analgesic represented by morphine or against addiction; analgesic enhancer; antiobestic or appetite suppressors; treating or prophylactic agents for cognitive impairment and dementia/ amnesia in aging, cerebrovascular diseases and Alzheimer's disease; agents for treating developmental cognitive abnormality in attention deficit, hyperactivity disorder and learning disability; remedy for schizophrenia; agents for treating neurodegenerative diseases represented by Parkinsonism and chorea; anti-depressants or treating agents for affective disorder; treating or prophylactic agents for diabetes insipidus;

treating or prophylactic agents for polyuria; or remedy for hypotension; their administration dosage or frequency can be varied depending on gender, age, body weight, degree of symptoms of individual patients and kind and extent of intended therapeutic effect. In general terms, the dose can normally range from 0.001 to 50 mg per day per kilogram of body weight, which can be administered at a time or by plural times. Preferably the dose is within a range of from about 0.01 to about 25 mg/kg per day, in particular, from about 0.05 to about 10 mg/kg per day.

Examples

Hereinafter the present invention is explained more specifically, referring to working Examples. Unless otherwise specified, those various reagents used in the working Examples were those available on the market, and, H-NMR values were measured, using tetramethylsilane as the reference material, using AL-400-2(400MHz, JEOL Co). Also the mass spectra were measured with Micromass ZQ (Waters Co.), by electro spray ionizing method (ESI) or atomspehric pressure chemical ionization method (APCI).

Production Example 1

Production of spiro[4.5]decane-6-carbaldehyde

1) Spiro[4.5]decan-6-one

Cyclohexanone (3.0 mL) was dissolved in toluene (60 mL), and cooled to 0°C in nitrogen atmosphere. Potassium tert-butoxide (6.86 g) was added to the reaction liquid at 0°C and stirred for 30 minutes. To the resulting suspension, 1,4-dibromomethane (3.65 mL) was added, and then the reaction liquid was stirred at 150°C for 6 hours. Cooling the reaction liquid to room temperature, water was added thereto, followed by extraction with ethyl acetate. The extract was dried over anhydrous magnesium sulfate, removed of the solvent by distillation, and the resulting residue was purified on silica gel column chromatography (hexane/ethyl acetate = 50/1) to provide 690.0 mg of the title compound as a colorless, oily substance.

2) Spiro[4.5]decane-6-carbaldehyde

A solution of diethyl(isocyanomethyl)phosphonate (410 μL) in diethyl ether (5 mL) was cooled to -78°C in nitrogen atmosphere. After addition of 1.54M n-butyl lithium solution in hexane (1.7 mL) to the reaction liquid at -78°C, the temperature was raised to 0°C and stirred for 15 minutes. To the resulting solution spiro[4.5]decan-6-one (300 mg) was added at 0°C, and the temperature was raised to room temperature under stirring. An hour thereafter, conc. hydrochloric acid (5 mL) was added to the reaction liquid at room temperature, followed by further 10 hours' stirring. The resulting solution was diluted with water and extracted with diethyl ether. The extract was dried over anhydrous magnesium sulfate, and from which the solvent was distilled off to provide the title compound in crude, unpurified form, as a colorless oily substance.

Production Example 2

Preparation of spiro[3.5]nonane-5-carbaldehyde

1) Ethyl 1,4-dioxaspiro[4.5]decane-6-carboxylate

Ethyl 2-oxocyclohexane carboxylate (11.3 g) and ethylene glycol (11 mL) were dissolved in toluene (100 mL). To the reaction liquid camphorsulfonic acid (1.03g) was added and refluxed for 8 hours with Dean-Stark apparatus. The reaction liquid was cooled to room temperature, diluted with diethyl ether and washed with saturated aqueous sodium hydrogencarbonate solution. The extract was washed with saturated brine, dried over anhydrous magnesium sulfate, and from which the solvent was distilled off to provide a crude product of the title compound.

2) 1,4-Dioxaspiro[4.5]dec-6-ylmethanol

The compound as obtained in 1) was dissolved in tetrahydrofuran (120 mL) and cooled to 0°C in nitrogen atmosphere. To the reaction liquid lithium aluminum hydride (3.06 g) was added at 0°C, and then the temperature was raised to room temperature, followed by an overnight stirring. The reaction liquid was again cooled to 0°C, to which sodium sulfate decahydrate was added and stirred for an hour, followed by drying by addition of anhydrous magnesium sulfate and filtering the insoluble matter off. Distilling off the solvent in the filterate, 9.80 g of a crude product of the title

compound was obtained.

3) 6-[(Benzyloxy)methyl]-1,4-dioxaspiro[4.5]decane

The compound (9.80 g) as obtained in 2) was dissolved in tetrahydrofuran (100 mL) and cooled to 0°C in nitrogen atmosphere. To the reaction liquid 60 - 72 % sodium hydride (in the form of an oil dispersion) (3.34 g) was added at 0°C, followed by 30 minutes' stirring. To the resulting reaction liquid, benzyl bromide (8.4 mL) was added at 0°C, the temperature was raised to room temperature, and the system was stirred for 3 hours. The reaction liquid was diluted with diethyl ether, and washed first with water and successively, with saturated brine. The organic layer was dried over anhydrous magnesium sulfate, the solvent was distilled off, and the resulting residue was purified on silica gel column chromatography (hexane/ethyl acetate = 19/1) to provide 7.24 g of the title compound.

4) 2-[(Benzyloxy)methyl]cyclohexanone

The compound (3.37 g) as obtained in 3) was dissolved in tetrahydrofuran (30 mL), to which 10% hydrochloric acid (10 mL) was added at room temperature, followed by 3 hours' stirring. The reaction liquid was diluted with diethyl ether and washed first with water, successively with saturated aqueous sodium hydrogencarbonate solution and then with saturated brine. The organic layer was dried over anhydrous magnesium sulfate, the solvent was distilled off, and 2.94 g of a crude product of the title compound was obtained.

5) 2-[(Benzyloxy)methyl]-1-[1-(phenylthio)cyclopropyl]cyclohexanol

A cyclopropyl phenyl sulfide (2.33 mL) solution in tetrahydrofuran (50 mL) was cooled to 0°C in nitrogen atmosphere. To the reaction liquid, 1.0 M n-butyl lithium hexane solution (16 mL) was added at 0°C, followed by an hour's stirring. The reaction liquid was cooled to -78°C, and to which a tetrahydrofuran solution (10 mL) of the compound (2.94 g) as obtained in 4) was added at -78°C, followed by stirring at -78°C for 30 minutes and then at 0°C for an hour. Water was added to the reaction liquid which then was extracted with diethyl ether. The extract was washed first with water and then with saturated brine, dried over anhydrous magnesium sulfate, removed of the solvent by distillation, and the resulting residue was purified on silica gel column chromatography (hexane/ethyl acetate = 19/1) to provide 3.04 g of the title compound.

6) 5-[(Benzyloxy)methyl]spiro[3.5]nonan-1-one

The compound (3.04 g) as obtained in 5) was dissolved in toluene (40 mL), to which p-toluenesulfonic acid monohydrate (1.60 g) and water (0.15 mL) were added and stirred at 90°C for 5 hours. The reaction liquid was diluted with diethyl ether and washed with water, 10% aqueous sodium hydroxide solution and saturated brine, by the order stated. The organic layer was dried over anhydrous magnesium sulfate, removed of the solvent by distillation, and the resulting residue was purified on silica gel column chromatography (hexane/ethyl acetate = 19/1) to provide 670 mg of the title compound.

7) 5-[(Benzyloxy)methyl]spiro[3.5]nonane

The compound (670 mg) as obtained in 6) was dissolved in diethylene glycol (3 mL), to which hydrazine monohydrate (1.5 mL) and potassium carbonate (838 mg) were added, followed by stirring under heating at 150°C for 3 hours and at 200°C for 5 hours. The reaction liquid was cooled to room temperature, diluted with diethyl ether, and then washed with 10% hydrochloric acid and then with saturated brine. The organic layer was dried over anhydrous magnesium sulfate and removed of the solvent by distillation, to provide 224 mg of a crude title compound.

8) Spiro[3.5]non-5-ylmethanol

The compound (224 mg) as obtained in 7) was dissolved in methanol (5 mL), to which a catalytic amount of activated carbon-carried palladium hydroxide was added, and the system was stirred at room temperature for 4 hours in hydrogen atmosphere of one atmospheric pressure. Filtering off the insoluble matter with Celite®, the filtrate was condensed to provide 151 mg of crude title compound.

9) Spiro[3.5]nonane-5-carbaldehyde

The compound (151 mg) as obtained in 8) was dissolved in dimethylsulfoxide (5 mL) and to which triethylamine

(2 mL) and anhydrous sulfurylic acid-pyridine complex (1.17 g) were added, followed by an hour's stirring at room temperature. The reaction liquid was diluted with diethyl ether and washed successively with water, 10% hydrochloric acid, saturated aqueous sodium hydrogen- carbonate solution and saturated brine. The organic layer was dried over anhydrous magnesium sulfate and distilled off the solvent to provide 120 mg of crude title compound.

Production Example 3

Preparation of spiro[2.5]octane-4-carbaldehyde

1) Ethyl 1,4-dioxaspiro[4.5]dec-6-yl acetate

Ethyl (2-oxocyclohexyl)acetate (50.05 g) and ethylene glycol (45.5 mL) were dissolved in toluene (200 mL). p-Toluenesulfonic acid monohydrate (7.75 g) was added to the reaction liquid which then was refluxed for 5 hours using Dean-Stark apparatus. The reaction liquid was cooled to room temperature and saturated aqueous sodium hydrogencarbonate solution was added thereto, followed by extraction with ethyl acetate. The extract was washed with saturated brine, dried over anhydrous magnesium sulfate and removed of the solvent by distillation to provide 76.54 g of crude title compound as a pale, yellowish brown oily substance.

2) 2-(1,4-Dioxaspiro[4.5]-dec-6-yl)ethanol

The compound (76.54 g) as obtained in 1) was dissolved in tetrahydrofuran (350 mL) and cooled to 0°C in nitrogen atmosphere. To the reaction liquid lithium aluminum hydride (10.36 g) was added at 0°C, followed by 3 hours' stirring. To the resulting reaction liquid sodium sulfate decahydrate (51.85 g) was added and stirred at room temperature for an overnight. The insoluble matter in the solution was filtered off with Celite®, and the filtrate was condensed to provide crude title compound (63.77 g) as a pale yellow, oily substance.

3) 2-(2-Chloroethyl)cyclohexanone

The compound (63.77 g) as obtained in 2) was dissolved in acetonitrile (40 mL) and added to conc. hydrochloric acid (250 mL) which had been cooled to 0°C. The reaction liquid's temperature was raised to room temperature, followed by 1.5 hours' heating under reflux. The reaction liquid was cooled to room temperature, diluted with water and extracted with hexane. The extract was successively washed with water and then with saturated aqueous sodium hydrogencarbonate solution, dried over anhydrous sodium sulfate and removed of the solvent by distillation to provide crude title compound (46.15 g) as a pale yellow, oily substance.

4) Spiro [2.5]octan-4-one

The compound (46.15 g) as obtained in 3) was dissolved in ethanol (100 mL) and cooled to 0°C. To the reaction liquid powdery potassium hydroxide (19.97 g) was added at 0°C and the temperature was raised to room temperature, followed by 3 hours' stirring. Potassium chloride as precipitated was filtered off, and the filterate was used in the subsequent reaction as an ethanol solution of the title compound.

5) Spiro[2.5]octane-4-carbonitrile

Potassium tert-butoxide (158.0 g) was suspended in dimethylsulfoxide (370 mL), and into which p-toluenesulfonylmethyl isocyanide (60.60 g) was added under cooling with ice, followed by 15 minutes' stirring at 0°C. Into the resulting brown-colored reaction liquid, an ethanol solution of the spiro[2.5]octan-4-one as obtained in 4) was added at 0°C, heated to room temperature and stirred for 3 hours. To the reaction liquid, water (300 mL) and hexane (300 mL) were added, followed by addition of 10% hydrochloric acid (400 mL). The reaction liquid was extracted with hexane, and the extract was washed with water and saturated aqueous solution of sodium hydrogencarbonate. The organic layer was dried over anhydrous sodium sulfate, from which the solvent was distilled off, and the resulting residue was purified on silica gel column chromatography (hexane/ethyl acetate = 3/1) to provide 39.95 g of the title compound as an yellowish brown, oily substance.

6) Spiro[2.5]octane-4-carbaldehyde

The compound (39.95 g) as obtained in 5) was dissolved in hexane (250 mL), and cooled to 0°C in nitrogen atmosphere. To the reaction liquid, 0.95 M diisobutylaluminum hydride-in-hexane solution (400 mL) was dropped at 0°C.

After the end of dropping, the reaction liquid was heated to room temperature and stirred for 3 hours. The resulting reaction liquid was again cooled to 0°C and into which 10% hydrochloric acid (300 mL) was dropped, followed by an hour's stirring at room temperature. The reaction liquid was extracted with hexane, and to the hexane extract first water (1.5 L) and then sodium hydrogensulfite (500 g) were added, followed by violent stirring at room temperature to extract the title compound as its hydrogen sulfite adduct in a water layer. The aqueous layer as extracted was separated, and to which methyl tert-butyl ether (1L) was added, followed by addition of sodium hydroxide (280 g) and violent stirring at room temperature. Separating the ether layer, the aqueous layer was again extracted with methyl tert-butyl ether. The resulting ether layers were combined and dried over anhydrous magnesium sulfate, and from which the solvent was distilled off to provide 20.04 g of the title compound as a pale yellow, oily substance.

Production Example 4

Preparation of spiro[bicyclo[2.2.1]heptane-2,1'-cyclopropane]-3-carbaldehyde

1) Spiro[bicyclo[2.2.1]heptane-2,1'-cyclopropane]-3-one

Zinc powder (15.10 g) was suspended in diethyl ether (70 mL) and to which cuprous chloride (2.29 g) was added at room temperature, followed by 30 minutes' refluxing in nitrogen atmosphere. The suspension was cooled to 0°C and to which 3-methylenebicyclo[2.2.1]heptan-2-one (7.0 mL) was added at 0°C. Into the reaction liquid, methane diiodide (7.0 mL) was slowly dropped at 0°C and after the end of the dropping, the reaction liquid was refluxed for 30 hours in nitrogen atmosphere. Cooling the reaction liquid to room temperature, the insoluble matter was filtered off with Celite® and the filterate was washed twice with 5% aqueous sodium thiosulfate solution. The organic layer was dried over anhydrous magnesium sulfate, removed of the solvent by distillation, and the resulting residue was purified on silica gel column chromatography (hexane/ethyl acetate = 100/1-7/1) to provide 4.89 g of the title compound as a colorless oily substance.

2)Spiro[bicyclo[2.2.1]heptane-2,1'-cyclopropane]-3-carbaldehyde

Methoxymethyltriphenylphosphonium chloride (6.41 g) was suspended in tetrahydrofuran (80 mL) and cooled to 0°C in nitrogen atmosphere. After adding 1.56 M n-butyl lithium-in-hexane solution (36.2 mL) at 0°C, the reaction liquid was stirred for an hour. Into the resulting deep red-colored solution, a spiro[bicyclo[2.2.1]heptane-2,1'-cyclopropane]-3-one (6.41 g) solution in tetrahydrofuran(10 mL) was dropped at 0°C, and thereafter the temperature was raised to room temperature, followed by an overnight's stirring. To the reaction liquid, 5M hydrochloric acid (50 mL) was added at room temperature and stirred for further 3 hours. The reaction liquid was diluted with water and extracted with diethyl ether. The extract was washed with saturated brine, dried over anhydrous sodium sulfate, removed of the solvent by distillation and the resulting residue was purified on silica gel column chromatography (hexane/ethyl acetate = 99/1 - 97/3) to provide 5.04 g of the title compound as a colorless oily substance.

Production Example 5

Preparation of 2,2-dimethyl-1,3-dioxolan-5-yl methanesulfonate

1) 2,2-Dimethyl-1,3-dioxolan-5-ol

A 2,2-dimethyl-1,3-dioxolan-5-one (930 mg) solution in tetrahydrofuran (10 mL) was cooled to 0°C in nitrogen atmosphere. To the reaction liquid lithium aluminium hydride (293 mg) was added at 0°C, followed by 30 minutes' stirring. After adding sodium sulfate decahydrate (3 g) to the reaction liquid at 0°C, the temperature was raised to room temperature, followed by further 2 hours' stirring. The insoluble matter was filtered off and the filterate was condensed. The resulting residue was purified on silica gel column chromatography (hexane/ethyl acetate = 3/2) to provide 743 mg of the title compound as a colorless, oily substance.

2) 2,2-Dimethyl-1,3-dioxolan-5-yl methanesulfonate

A2,2-dimethyl-1,3-dioxolan-5-ol (743 mg) solution in tetrahydrofuran (10 mL) was cooled to 0°C in nitrogen atmosphere. To the reaction liquid, triethylamine (1.87 mL) and methanesulfonyl chloride (520 μL) were successively added at 0°C, followed by 30 minutes' stirring. After addition of saturated aqueous ammonium chloride solution, the reaction liquid was extracted with ethyl acetate. The extract was washed with saturated brine, dried over anhydrous magnesium sulfate and removed of the solvent by distillation to provide 837 mg of crude title compound as a colorless solid.

Production Example 6

Preparation of [(4R or 4S)-2,2,4-trimethyl-1,3-dioxolan-4-yl]methyl 4-methylbenzenesulfonate

Two kinds of optical isomers of (2,2,4-trimethyl-1,3-dioxolan-4-yl)methyl 4-methylbenzenesulfonate (976 mg) which is a per se known substance, was separated using CHIRALPAK® AD (Daicel Co., Ltd., 2 cmϕ × 25 cm; hexane/ethanol = 9/1), to provide as the first eluate 478 mg of (4S or 4R) body of the title compound, and as the second eluate, 477 mg of (4R or 4S) body of the title compound.

Example 1

Preparation of 1-[(2R)-2,3-dihydroxypropyl]-3-{1-[(6S or 6R)-spiro[4.5]dec-6-ylmethyl]piperidin-4-yl}-1,3-dihydro-2H-benzimidazol-2-one

1) 3-[(1-(spiro[4.5]dec-6-ylmethyl)piperidin-4-yl)-1,3-dihydro-2H-benzimidazol-2-one

To a 1-piperidin-4-yl-1,3-dihydro-2H-benzimidazol-2-one (360 mg) solution in dichloromethane (15 mL), the compound (250 mg) as obtained in Production Example 1 was added at room temperature, followed by addition of sodium triacetoxyborohydride (380 mg) at room temperature. The mixed solution was stirred at room temperature for 3 hours, 1M aqueous sodium hydroxide solution was added, and the reaction liquid was extracted with chloroform. The extract was washed with saturated brine, dried over anhydrous magnesium sulfate, removed of the solvent by distillation, and the resulting residue was purified on silica gel column chromatography (chloroform/methanol = 50/1 - 30/1) to provide 179.5 mg of the title compound as a colorless solid.

2) 1-{[(4R)-2,2-dimethyl-1,3-dioxolan-4-yl]methyl}-3-[1-(spiro[4.5]-dec-6-ylmethyl)piperidin-4-yl]-1,3-dihydro-2H-benzimidazol-2-one

The compound (90 mg) as obtained in 1) was dissolved in dimethylformamide (3 mL), and to the solution 60 - 72% sodium hydride (oil dispersion) (20 mg) was added at room temperature, followed by 30 minutes' stirring. After addition of [(4S)-2,2-dimethyl-1,3-dioxolan-4-yl]methyl 4-methylbenzene- sulfonate (140 mg) and potassium iodide (20 mg), the reaction liquid was stirred at 60°C for 14 hours. The reaction liquid was cooled to room temperature, to which 1 M aqueous sodium hydroxide solution was added, followed by extraction with ethyl acetate. The extract was washed with saturated brine, dried over anhydrous magnesium sulfate, removed of the solvent by distillation, and the resulting residue was purified on silica gel column chromatography (hexane/ethyl acetate = 5/1 - 2/1) to provide 100.2 mg of the title compound as a pale yellow, oily substance.

3) 1-{[(4R)-2,2-dimethyl-1,3-dioxolan-4-yl]methyl}-3-{1-[(6S or 6R)-spiro[4.5]-dec-6-ylmethyl)piperidin-4-yl]-1,3-dihydro-2H-benzimidazol-2-one

The two kinds of diastereomers of the compound (100.2 mg) as obtained in 2) were separated using CHIRALPAK® AD (Daicel Co., Ltd.; 2 cmϕ × 25 cm, hexane/2-propanol/diethylamine = 6/1/0.007), to provide as the first eluate 44.1 mg of (6S or 6R) body of the title compound.

4) 1-[(2R)-2,3-dihydroxypropyl]-3-{1-[(6S or 6R)-spiro[4.5]dec-6-ylmethyl]piperidin-4-yl}-1,3-dihydro-2H-benzimidazol-2-one

The compound (44 mg) as obtained in 3) was dissolved in tetrahydrofuran (2 mL), to which 5M hydrochloric acid (2 mL) was added at room temperature and stirred for an hour. The resulting reaction solution was cooled to 0°C, neutralized with 1M aqueous sodium hydroxide solution and extracted with ethyl acetate. The extract was washed with saturated brine, dried over anhydrous magnesium sulfate, removed of the solvent by distillation and the resulting residue was purified on silica gel preparative thin layer chromatography (chloroform/methanol = 20/1) to provide 28.8 mg of the title compound as a colorless oily substance.

$^1$H-NMR($CDCl_3$)δ=1.14-1.85(19H,m),1.88-1.99(1H,m),
2.14-2.55(5H,m),2.90-2.99(1H,m),3.05-3.14(1H,m),
3.55-3.62(2H,m),3.96-4.10(3H,m),4.26-4.40(1H,m),
7.07-7.15(3H,m),7.28-7.34(1H,m)
ESI-MS(+20eV) m/z 442.2

Example 2

Preparation of 1-[(2R)-2,3-dihydroxypropyl]-3-{1-[(4S)-spiro[2.5]oct-4-ylmethyl]piperidin-4-yl}-1,3-dihydro-2H-benzimidazol-2-one

1) 3-[1-(spiro[2.5]oct-4-ylmethyl)piperidin-4-yl]-1,3-dihydro-2H-benzimidazol-2-one

To 1-piperidin-4-yl-1,3-dihydro-2H-benzimidazol-2-one (4.00 g) solution in tetrahydrofuran (150 mL), the compound (3.02 g) as obtained in Production Example 3 was added at room temperature, and successively, sodium triacetoxyborohydride (4.68 g), at room temperature. The mixed solution was stirred at room temperature for 2 hours, followed by addition of 1.5M aqueous sodium hydroxide solution and extraction with chloroform. The extract was dried over anhydrous magnesium sulfate and removed of the solvent by distillation to provide 4.98 g of crude title compound as a pale, yellowish brown solid.

2) 1-(Methylsulfonyl)-3-[1-(spiro[2.5]oct-4-ylmethyl)piperidin-4-yl]-1,3-dihydro-2H-benzimidazol-2-one

The compound (4.98 g) as obtained in 1) was dissolved in chloroform (150 mL). To the solution triethylamine (7 mL) and methanesulfonyl chloride (1.94 mL) were added at room temperature, followed by 2 hours' stirring at room temperature. The reaction liquid was diluted with chloroform and washed with saturated aqueous sodium hydrogencarbonate solution. The organic layer was dried over anhydrous magnesium sulfate, removed of the solvent by distillation and the resulting residue was purified on silica gel column chromatography (chloroform/methanol = 100/1-15/1) to provide 5.47 g of the title compound as a pale yellow, oily substance.

3) 1-(Methylsulfonyl)-3-{1-[(4S)-spiro[2.5]oct-4-ylmethyl)piperidin -4-yl}-1,3-dihydro-2H-benzimidazol-2-one

The two kinds of diastereomers of the compound (5.47 g) as obtained in 2) were separated using CHIRALPAK® AD (Daicel Co., Ltd.; 2 cmϕ × 25 cm, hexane/ethanol/diethylamine = 4/1/0.005) to provide as the first eluate 2.15g of (4S) body of the title compound.

4) 3-{1-[(4S)-spiro[2.5]oct-4-ylmethyl]piperidin-4-yl}-1,3-dihydro-2H-benzimidazol-2-one

The compound (2.15 g) as obtained in 3) was dissolved in tetrahydrofuran (70 mL), to which 1M tetra-n-butylammonium fluoride-in-tetrahydrofuran solution (9.5 mL) was added at room temperature and stirred for 3 hours. Saturated aqueous sodium hydrogencarbonate solution was added to the reaction liquid which then was extracted with ethyl acetate. The extract was washed with saturated brine, dried over anhydrous magnesium sulfate, removed of the solvent by distillation and the resulting residue was purified on N basic silica gel column chromatography (hexane/ethyl acetate = 1/1-1/2) to provide 1.56 g of the title compound as a colorless solid.

5)1-{[(4R)-2,2-dimethyl-1,3-dioxolan-4-yl]methyl}-3-{1-[(4S)-spiro-[2.5]-oct-4-ylmethyl]piperidin-4-yl}-1,3-dihydro-2H-benzimidazol-2-one

3-{1-[(4S)-spiro[2.5]oct-4-ylmethyl]piperidin-4-yl}-1.3-dihydro-2H-benzimidazol-2-one (2.94 g) was dissolved in dimethylformamide (60 mL), and to which 60-72% sodium hydride (oil dispersion) (706.6 mg) was added at room temperature and stirred for 30 minutes. To the reaction liquid, [(4S)-2,2-dimethyl-1,3-dioxolan-4-yl]methyl 4-methylbenzene- sulfonate (7.25 g) was added and stirred at 80°C for 7 hours. After cooling the reaction liquid to room temperature, water was added, followed by extraction with ethyl acetate. The extract was washed with saturated brine, dried over anhydrous magnesium sulfate, removed of the solvent by distillation and the resulting residue was purified on silica gel column chromatography (hexane/ethyl acetate = 2/1-1/2) to provide 3.43 g of the title compound as a pale yellow, oily substance.

6) 1-[(2R)-2,3-dihydroxypropyl]-3-{1-[(4S)-spiro[2.5]oct-4-ylmethyl]-piperidin-4-yl}-1,3-dihydro-2H-benzimidazol-2-one hydrochloride

The compound (2.71 g) as obtained in 5) was dissolved in methanol (15 mL), to which 10% hydrogen chloride/methanol solution (65 mL) was added and stirred for an overnight at room temperature. The reaction liquid was condensed, and the resulting solid was washed with ethanol to provide 1.60 g of the title compound as a colorless solid.

$^1$H-NMR($CDCl_3$)δ=0.31-0.42(2H,m),0.42-0.58(2H,m),

0.76-0.86(1H,m),1.40-1.75(4H,m),1.75-2.10(4H,m),
2.72-3.00(4H,m),3.22-3.42(3H,m),3.52-3.64(3H,m),
3.65-3.78(2H,m),4.00-4.08(3H,m),4.65-4.80(1H,m),
7.10-7.20(3H,m),8.02-8.08(1H,m),12.60(1H,brs)
ESI-MS(+20eV) m/z 414.4

Example 3

Preparation of 1-[2-hydroxy-1-(hydroxymethyl)ethyl]-3{1-[(1R,3S, 4S)-spiro[bicyclo[2.2.1]heptane-2,1'-cyclopropan-3-ylmethyl]-piperidin-4-yl}-1,3-dihydro-2H-benzimidazol-2-one

1) 3-[1-(Spiro[bicyco[2.2.1]heptane-2,1'-cyclopropan]-3-ylmethyl)-piperidin-4-yl]-1,3-dihydro-2H-benzimidazol-2-one

To 1-piperidin-4-yl-1,3-dihydro-2H-benzimidazol-2-one (1.5 g) solution in tetrahydrofuran (15 mL), the compound (1.2 g) as obtained in Production Example 4 was added at room temperature, and successively sodium triacetoxyborohydride (2.0 g) was added and stirred for 2 days at room temperature. To the reaction liquid 1M aqueous sodium hydroxide solution was added, followed by extraction with ethyl acetate. The extract was dried over anhydrous magnesium sulfate, then the solvent was distilled off and the resulting residue was purified on silica gel column chromatography (chloroform/methanol = 100/1 -10/1) to provide 1.53 g of the title compound as a pale orange-colored solid.

2) 1-(Methylsulfonyl)-3-[1-(spiro[bicyclo[2.2.1]heptane-2,1'-cyclopropan]-3-ylmethyl)piperidin-4-yl]-1,3-dihydro-2H-benzimidazol-2-one

The compound (1.53 g) as obtained in 1) was dissolved in chloroform (15 mL) and cooled to 0°C. To the solution first triethylamine (1.2 mL) and successively methanesulfonyl chloride (512 μL) were added at 0°C, followed by an hour's stirring at 0°C. The reaction liquid was diluted with ethyl acetate and washed with 1M aqueous sodium hydroxide solution. The organic layer was dried over anhydrous magnesium sulfate and removed of the solvent by distillation. The resulting residue was purified on silica gel column chromatography (chloroform/methanol = 200/1 - 20/1) to provide 1.90 g of the title compound as a pale yellow, oily substance.

3) 1-(Methylsulfonyl)-3-{1-[(1R, 3S, 4S)-spiro[bicyclo[2.2.1]heptane-2,1'-cyclopropan]-3-ylmethyl]piperidin-4-yl}-1,3-dihydro-2H-benzimidazol-2-one

The four kinds of optical isomers of the compound (1.90 g) as obtained in 2) were separated using CHIRALPAK® AD (Daicel Co., Ltd.; 2 cmϕ × 25 cm, hexane/ethanol/diethylamine = 9/1/0.01), to provide as the third eluate 420 mg of the title compound (1R, 3S, 4S) bodies.

4) 3-{1-[(1R, 3S, 4S)-spiro[bicyclo[2.2.1]heptane-2,1'-cyclopropan]-3-ylmethyl]piperidin-4-yl}-1,3-dihydro-2H-benzimidazol-2-one

The third eluate (420 mg) as obtained in 3) was dissolved in tetrahydrofuran (5 mL), and to which 1 M tetra-n-butylammonium fluoride solution in tetrahydrofuran (1.5 mL) was added at room temperature and stirred for an overnight. After distilling off the solvent from the reaction liquid, the resulting residue was purified on silica gel column chromatography (chloroform/methanol = 100/1-10/1) to provide 294 mg of the title compound as a colorless solid.

5) 1-(2,2-Dimethyl-1,3-dioxolan-5-yl)-3-{1-[(1R,3S, 4S)-spiro-[bicyclo[2.2.1]heptane-2,1'-cyclopropan]-3-ylmethyl]piperidin-4-yl; - 1,3-dihydro-2H-benzimidazol-2-one

The compound (294 mg) as obtained in 4) above was dissolved in dimethylformamide (5 mL), and to which 60 - 72% sodium hydride (an oil dispersion) (100 mg) was added at room temperature and stirred for 30 minutes. To the resulting reaction liquid the compound (353 mg) as obtained in Production Example 5 was added and stirred at 120°C for 2 hours. Thereafter 60 - 72% sodium hydride (an oil dispersion) (100 mg) and the compound (353 mg) as obtained in Production Example 5 was added to the reaction liquid twice at an interval of an hour. The reaction liquid was cooled to room temperature, to which 1M aqueous sodium hydroxide solution was added and extracted with ethyl acetate. The extract was washed with saturated brine, dried over anhydrous magnesium sulfate, removed of the solvent by distillation and the resulting residue was purified on silica gel column chromatography (chloroform/methanol = 400/1 - 20/1) to provide 132 mg of the title compound as a pale yellow, oily substance.

6) 1-[2-hydroxy-1-(hydroxymethyl)ethyl]-3-{1-[(1R, 3S, 4S)-spiro-bicyclo[2.2.1]heptane -2,1'-cyclopropan]-3-ylmethyl] piperidin-4-yl}-1,3-dihydro-2H-benzimidazol-2-one hydrochloride

The compound (132 mg) as obtained in 5) was dissolved in methanol (5 mL), to which 6M hydrochloric acid (100 μL) was added at room temperature and stirred for 3 hours. Distilling the solvent off from the reaction liquid, the resulting solid was washed with ethyl acetate to provide 128 mg of the title compound as a colorless solid.

$^1$H-NMR($CD_3OD$)δ=0.29-0.39(2H,m),0.46-0.55(1H,m), 0.66-0.74(1H,m),1.46-1.68(6H,m),1.79-1.86(1H,m), 2.00-2.12(2H,m),2.28-2.37(1H,m),2.53-2.59(H,m), 2.74-3.26(5H,m),3.49-3.62(1H,m),3.71-3.82(2H,m), 3.94(2H,dd,J=5.3,11.6Hz),4.08(2H,dd,J=8.1,11.6Hz), 4.45-4.65(2H,m),7.06-7.14(2H,m),7.28-7.41(2H,m)
ESI-MS(+20eV) m/z 426.2

Example 4

Preparation of [(2S or 2R)-2,3-dihydroxy-2-methylpropyl]-3-{1-[(4S)-spiro[2.5]oct-4-ylmethyl]piperidin-4-yl}-1,3-dihydro-2H-benzimidzol-2-one (2R,

3R)-3-carboxy-2,3-dihydroxypropionic acid salt

1) 1-{1-[(4S)-spiro[2.5]oct-4-ylmethyl]piperidin-4-yl}-3-{[(4S or 4R)-2,2,4-trimethyl-1,3-dioxolan-4-yl]methyl}-1,3-dihydro-2H-benzimidazol-2-one

3-{1-[(4S)-spiro[2.5]oct-4-ylmethyl]piperidin-4-yl}-1,3-dihydro-2H-benzimidazol-2-one (753 mg) as obtained in Example 2 -4) and potassium iodide (372 mg) were dissolved in dimethylformamide (100 mL), and to which 60 - 72% sodium hydride (an oil dispersion) (428 mg) was added at room temperature and stirred for 30 minutes. To the resulting reaction liquid, a solution of the (4R or 4S)-2,2,4-trimethyl-1,3-dioxolan-4-yl]methyl 4-methylbenzenesulfonate (1.99 g), as obtained in Production Example 6 as the second eluate, in dimethylformamide (30 mL) was added at room temperature and stirred at 120°C for an overnight. To the reaction liquid saturated aqueous ammonium chloride solution was added, followed by extraction with ethyl acetate. The extract was washed with saturated aqueous sodium hydrogen- carbonate solution, dried over anhydrous magnesium sulfate, removed of the solvent by distillation and the resulting residue was purified on basic silica gel column chromatography (hexane/ethyl acetate = 10/1 - 5/1) to provide 1.27 g of the title compound as a pale yellow, oily substance.

2) 1-[(2S or 2R)-2.3-dihydroxy-2-methylpropyl]-3-{1-[(4S)-spiro-[2.5]oct-4-ylmethyl]piperidin-4-yl}-1,3-dihydro-2H-benzimidazol-2-one (2R, 3R)-3-carboxy-2,3-dihydroxypropionic acid salt

The compound (1.27 g) as obtained in 1) was dissolved in tetrahydrofuran (20 mL), and to which 1 M hydrochloric acid (20 mL) was added at room temperature and stirred for an overnight. The resulting reaction liquid was cooled to 0°C, and to which saturated aqueous sodium hydrogencarbonate solution was added, followed by extraction with chloroform. The extract was dried over anhydrous magnesium sulfate, removed of the solvent by distillation and the resulting residue was purified on silica gel column chromatography (chloroform/methanol = 30/1 - 4/1) to provide 1.09 g of free amine body of the title compound. This free amine body (33.2 mg) and (2R, 3R)- tartaric acid (11.6 mg) were dissolved in methanol (3 mL), and the solvent was distilled off. The resulting solid was washed with ethyl acetate to provide 44.8 mg of the title compound as a colorless solid.

$^1$H-NMR($CD_3OD$)δ=0.35(2H,brs),0.45(2H,brs),0.84(1H,m), 1.14(1H,d,J=6.2Hz),1.20(3H,s),1.41-1.82(8H,m),2.01(2H,m), 2.78-3.25(5H,m),3.30-3.51(2H,m),3.69(2H,m),3.92(2H,s), 4.43(2H,s),4.60(1H,m),7.12(2H,m),7.36(2H,m)
ESI-MS(+20eV) m/z 428.3

Example 5

Preparation of 1-[(2R)-2,3-dihydroxypropyl]-3-{1-[(1R, 3S, 4S)-spiro[bicyclo-[2.2.1]hentane-2,1'-cyclopropan]-3-ylmethyl]piperidin-4-yl}-1,3-dihydro-2H-benzimi dazol-2-one hydrochloride

1) 1-{[(4R)-2,2-dimethyl-1,3-dioxolan-4-yl]methyl}-3-{1-[(1R, 3S, 4S)-spiro[bicyclo[2.2.1]heptane-2,1'-cyclopropan]-3-ylmethyl]piperidin-4-yl}-1,3-dihydro-2H-benzimidazol-2-one

3-{1-[(1R, 3S, 4S)-spiro[bicyclo[2.2.1]heptane-2,1'-cyclopropan]-3-ylmethyl]piperidin-4-yl}-1,3-dihydro-2H-benzimidazol-2-one (1.47g) as obtained in 4) of Example 3 was dissolved in dimethylformamide (25 mL), and to which 60 - 72% sodium hydride (an oil dispersion) (418 mg) was added at room temperature and stirred for 15 minutes. To the resulting reaction liquid, a [(4S)-2,2-dimethyl-1,3-dioxolan-4-yl]methyl 4-methylbenzenesulfonate (2.39 g) solution in dimethylformamide (5 mL) was added at room temperature, and stirred at 80°C for 5 hours. The reaction liquid was cooled to room temperature, to which phosphate buffer (pH 6.5) was added, followed by extraction with ethyl acetate. The extract was washed with water, dried over anhydrous sodium sulfate, removed of the solvent by distillation and the resulting residue was purified on silica gel column chromatography (hexane/ethyl acetate = 9/1 -1/1) to provide 1.80 g of the title compound as a colorless, oily substance.

2) 1-[(2R)-2,3-dihydroxypropyl]-3-{1-[(1R, 3S, 4S)-spiro[bicyclo[2.2.]heptane-2,1'-cycloropropan]-3-ylmethyl]piperidin-4-yl}-1,3-dihyro-2H-benzimi dazol-2-one hydrochloride

The compound (1.75 g) as obtained in 1) was dissolved in tetrahydrofuran (57 mL), to which 1M hydrochloric acid (19 mL) was added at room temperature and stirred for 30 hours. The reaction liquid was neutralized by addition of aqueous sodium hydrogencarbonate solution at 0°C, followed by extraction with ethyl acetate. The extract was washed with saturated brine, dried over anhydrous sodium sulfate, removed of the solvent by distillation and the resulting residue was purified on silica gel column chromatography (chloroform/methanol = 1/0 - 95/5) to provide 1.42 g of free amine body of the title compound. The amine body was dissolved in diethyl ether, to which 4M hydrogen chloride solution in dioxane (0.92 mL) was added at room temperature. Distilling the solvent off from the resulting suspension, the remaining solid was washed with ethanol/ethyl acetate (1/1) mixture to provide 1.06 g of the title compound as a colorless solid.

$^1$H-NMR($CD_3OD$)δ=0.27-0.38(2H,m),0.43-0.57(1H,m),
0.63-0.73(1H,m),1.40-1.69(6H,m),1.78-1.87(1H,m),
1.96-2.09(2H,m),2.27-2.37(1H,m),2.52-2.60(1H,m),
2.70-3.27(6H,m),3.51-3.60(2H,m),3.65-3.80(2H,m),
3.87-4.03(3H,m),4.52-4.67(1H,m),7.08-7.16(2H,m),
7.22-7.29(1H,m),7.32-7.41(1H,m)
ESI-MS(+20eV) m/z 426.2

Industrial Applicability

Those compounds of the invention have the action to inhibit binding of nociceptin to nociceptin receptor ORL1 and are useful as analgesic against diseases accompanied with pain such as cancerous pain, postoperative pain, migraine, gout, chronic rheumatism, chronic pain and neuralgia; relievers against tolerance to narcotic analgesic represented by morphine; relievers against dependence on narcotic analgesic represented by morphine or against addiction; analgesic enhancers; antiobestic or appetite suppressors; treating or prophylactic agents for cognitive impairment and dementia/ amnesia in aging, cerebrovascular diseases and Alzheimer's disease; agents for treating developmental cognitive abnormality in attention deficit, hyperactivity disorder and learning disability; remedy for schizophrenia; agents for treating neurodegenerative diseases represented by Parkinsonism and chorea; anti-depressant or treating agents for affective disorder; treating or prophylactic agents for diabetes insipidus; treating or prophylactic agents for polyuria; and remedy for hypotension and the like.

## Claims

**1.** A compound of the formula (I):

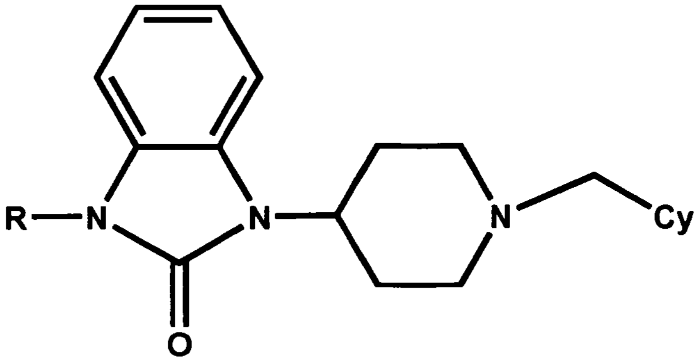

(I)

in which, R stands for a di-hydroxy-substituted $C_1$ - $C_6$ alkyl group, and Cy is a spiro[4.5]dec-6-yl, spiro[2.5]oct-4-yl, spiro[3.5]non-5-yl, 3,3-dimethylbicyclo[2.2.1]hept-2-yl or 1-spiro(bicyclo[2.2.1]heptane-2,1'cyclopropan)-3-yl group, or a pharmaceutically acceptable salt thereof.

**2.** The compound according to Claim 1 in which R is a di-hydroxyl-substituted $C_3$ - $C_4$ alkyl group.

**3.** The compound according to Claim 2, in which R is 2,3-dihydroxypropyl, 2-hydroxy-1-(hydroxymethyl)ethyl or 2,3-dihydroxy-2-methylpropyl.

**4.** The compound according to Claim 1, in which the compound represented by the formula (I) is selected from the group consisting of

1) 1-(2,3-dihydroxypropyl)-3-[1-(spiro[4.5]dec-6-ylmethyl)-piperidin-4-yl]-1,3-dihydro-2H-benzimidazol-2-one,
2) 1-(2,3-dihydroxypropyl)-3-[1-(spiro[2.5]oct-4-ylmethyl)-piperidin-4-yl]-1,3-dihydro-2H-benzimidazol-2-one,
3) 1-[2-hydroxy-1-(hydroxymethyl)ethyl]-3-[1-(spiro[bicyclo-[2.2.1]heptane-2,1'-cyclopropan]-3-ylmethyl)piperidin-4-yl]-1,3-dihydro-2H-benzimidazol-2-one,
4) 1-(2,3-dihydroxy-2-methylpropyl)-3-[1-(spiro[2.5]oct-4-ylmethyl)piperidin-4-yl]-1,3-dihydro-2H-benzimidazol-2-one, and
5) 1-(2,3-dihydroxypropyl)-3-[1-(spiro[bicyclo[2.2.1]heptane-2,1'-cyclopropan]3-ylmethyl)piperidin-4-yl]-1,3-dihydro-2H-benzimidazol-2-one, or a pharmaceutically acceptable salt thereof.

**5.** The compound according to Claim 1, in which the compound represented by the formula(I) is 1-(2,3-dihydroxypropyl)-3-[1-(spiro[4.5]dec-6-ylmethyl)piperidin-4-yl]-1,3-dihydro-2H-benzimidazol-2-one, or a pharmaceutically acceptable salt thereof.

**6.** The compound according to Claim 1, in which the compound represented by the formula (I) is 1-(2,3-dihydroxypropyl)-3-[1-(spiro[2.5]oct-4-ylmethyl)piperidin-4-yl]-1,3-dihydro-2H-benzimidazol-2-one, or a pharmaceutically acceptable salt thereof.

**7.** The compound according to Claim 1, in which the compound represented by the formula (I) is 1-[2-hydroxy-1-(hydroxymethyl)ethyl]-3-[1-(spiro-[bicyclo[2.2.1]heptane-2,1'-cyclopropan]-3-ylmethyl)piperidin-4-yl]-1,3-dihydro-2H-benzimidazol-2-one, or a pharmaceutically acceptable salt thereof.

**8.** The compound according to Claim 1, in which the compound represented by the formula (I) is 1-(2,3-dihydroxy-2-methylpropyl)-3-[1-(spiro[2.5]oct-4-ylmethyl)-piperidin-4-yl]-1,3-dihydro-2H-benzimidazol-2-one, or a pharmaceutically acceptable salt thereof.

**9.** The compound according to Claim 1, in which the compound represented by the formula (I) is 1-(2,3-dihydroxypropyl)-3-[1-(spiro[bicyclo[2.2.1]-heptane-2,1'-cyclopropan]-3-ylmethyl)piperidin-4-yl]-1,3-dihydro-2H-benzimidazol-2-one, or a pharmaceutically acceptable salt thereof.

**10.** A pharmaceutical composition comprising a pharmaceutically acceptable adjuvant and a compound according to any one of Claims 1-9 or a pharmaceutically acceptable salt thereof.

**11.** A method for the manufacture of a medicament for use as an analgesic; reliever against tolerance to narcotic analgesics; reliever against dependence on narcotic analgesics or against addiction; analgesic enhancer; antiobestic or appetite suppressor; agent for cognitive impairment and dementia/ amnesia in aging, cerebrovascular disease and Alzheimer's disease; agent for treating developmental cognitive abnormality in attention deficit, hyperactivity disorder and learning disability; remedy for schizophrenia; agent for treating neurodegenerative diseases represented by Parkinsonism and chorea; anti-depressant or treating agents for affective disorder; treating or prophylactic agent for diabetes insipidus; treating or prophylactic agent for polyuria; and remedy for hypotension; which comprises combining a compound according to any one of Claims 1-9 or a pharmaceutically acceptable salt thereof, and a pharmaceutically acceptable adjuvant.

**12.** A compound according to any of claims 1-9 for use in medicine.

**Patentansprüche**

**1.** Eine Verbindung der Formel (I):

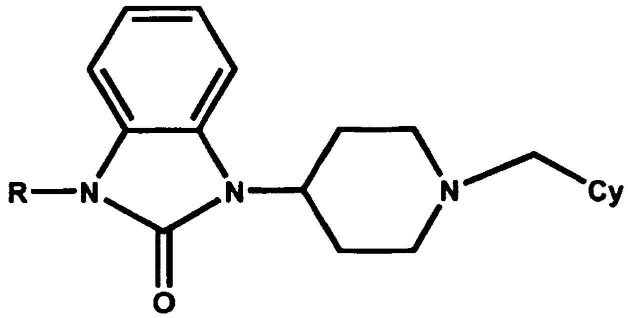

(I)

,

wobei R eine dihydroxysubstituierte $C_1$-$C_6$-Alkylgruppe bedeutet und Cy eine Spiro[4.5]dec-6-yl-, Spiro[2.5]oct-4-yl-, Spiro[3.5]non-5-yl-, 3,3-Dimethylbicyclo[2.2.1]hept-2-yl- oder 1-Spiro(bicyclo[2.2.1]-heptan-2,1'-cyclopropan)-3-yl-Gruppe ist, oder ein pharmazeutisch annehmbares Salz davon.

**2.** Die Verbindung gemäß Anspruch 1, wobei R eine dihydroxysubstituierte $C_3$-$C_4$-Alkylgruppe ist.

**3.** Die Verbindung gemäß Anspruch 2, wobei R 2,3-Dihydroxypropyl, 2-Hydroxy-1-(hydroxymethyl)ethyl oder 2,3-Dihydroxy-2-methylpropyl ist.

**4.** Die Verbindung gemäß Anspruch 1, wobei die durch die Formel (I) dargestellte Verbindung ausgewählt ist aus der Gruppe, bestehend aus

1) 1-(2,3-Dihydroxypropyl)-3-[1-(spiro[4.5]dec-6-ylmethyl)piperidin-4-yl]-1,3-dihydro-2H-benzimidazol-2-on,
2) 1-(2,3-Dihydroxypropyl)-3-[1-(spiro[2.5]oct-4-ylmethyl)piperidin-4-yl]-1,3-dihydro-2H-benzimidazol-2-on,
3) 1-[2-Hydroxy-1-(hydroxymethyl)ethyl]-3-[1-(spiro[bicyclo[2.2.1]heptan-2,1'-cyclopropan]-3-ylmethyl)piperidin-4-yl]-1,3-dihydro-2H-benzimidazol-2-on,
4) 1-(2,3-Dihydroxy-2-methylpropyl)-3-[1-(spiro[2.5]oct-4-ylmethyl)piperidin-4-yl]-1,3-dihydro-2H-benzimidazol-2-on und
5) 1-(2,3-Dihydroxypropyl)-3-[1-(spiro[bicyclo[2.2.1]heptan-2,1'-cyclopropan]-3-ylmethyl)piperidin-4-yl]-1,3-dihydro-2H-benzimidazol-2-on,
oder ein pharmazeutisch annehmbares Salz davon.

**5.** Die Verbindung gemäß Anspruch 1, wobei die durch die Formel (I) dargestellte Verbindung 1-(2,3-Dihydroxypropyl)-3-[1-(spiro[4.5]dec-6-ylmethyl)piperidin-4-yl]-1,3-dihydro-2H-benzimidazol-2-on ist, oder ein pharmazeutisch annehmbares Salz davon.

**6.** Die Verbindung gemäß Anspruch 1, wobei die durch die Formel (I) dargestellte Verbindung 1-(2,3-Dihydroxypropyl)-3-[1-(spiro[2.5]oct-4-ylmethyl)piperidin-4-yl]-1,3-dihydro-2H-benzimidazol-2-on ist, oder ein pharmazeutisch annehmbares Salz davon.

**7.** Die Verbindung gemäß Anspruch 1, wobei die durch die Formel (I) dargestellte Verbindung 1-[2-Hydroxy-1-(hydroxymethyl)ethyl]-3-[1-(spiro[bicyclo[2.2.1]heptan-2,1'-cyclopropan]-3-ylmethyl)piperidin-4-yl]-1,3-dihydro-2H-benzimidazol-2-on ist, oder ein pharmazeutisch annehmbares Salz davon.

**8.** Die Verbindung gemäß Anspruch 1, wobei die durch die Formel (I) dargestellte Verbindung 1-(2,3-Dihydroxy-2-methylpropyl)-3-[1-(spiro[2.5]oct-4-ylmethyl)piperidin-4-yl]-1,3-dihydro-2H-benzimidazol-2-on ist, oder ein pharmazeutisch annehmbares Salz davon.

**9.** Die Verbindung gemäß Anspruch 1, wobei die durch die Formel (I) dargestellte Verbindung 1-(2,3-Dihydroxypropyl)-3-[1-(spiro[bicyclo[2.2.1]heptan-2,1'-cyclopropan]-3-ylmethyl)piperidin-4-yl]-1,3-dihydro-2H-benzimidazol-2-on ist, oder ein pharmazeutisch annehmbares Salz davon.

**10.** Eine pharmazeutische Zusammensetzung, umfassend einen pharmazeutisch annehmbaren Hilfsstoff und eine Verbindung gemäß einem der Ansprüche 1-9 oder ein pharmazeutisch annehmbares Salz davon.

**11.** Ein Verfahren zur Herstellung eines Medikaments zur Verwendung als ein Analgetikum; Hilfsmittel gegen Betäubungsmitteltoleranz; Hilfsmittel gegen Betäubungsmittelabhängigkeit oder gegen Sucht; Verstärker für Analgetika; Mittel gegen Adipositas oder Appetitzügler; Mittel gegen kognitive Störung und Altersdemenz/Arnnesie, zerebrovaskuläre Erkrankung und Alzheimer-Krankheit; Mittel zur Behandlung von Hirnentwicklungsabnormalität bei Aprosexia, Hyperaktivitätsstörung und Lernschwäche; Heilmittel für Schizophrenie; Mittel zur Behandlung von neurodegenerativen Erkrankungen, wie z.B. Parkinson und Chorea; Antidepressivum oder Mittel zur Behandlung von Gemütskrankheit; Behandlungsmittel oder Prophylaktikum gegen Diabetes insipidus; Behandlungsmittel oder Prophylaktikum gegen Polyurie; und Heilmittel für Hypotonie; umfassend die Kombination einer Verbindung gemäß einem der Ansprüche 1-9 oder eines pharmazeutisch annehmbaren Salzes davon und eines pharmazeutisch annehmbaren Hilfsstoffs.

**12.** Eine Verbindung gemäß einem der Ansprüche 1-9 zur Verwendung in der Medizin.

## Revendications

**1.** Composé de la formule (I):

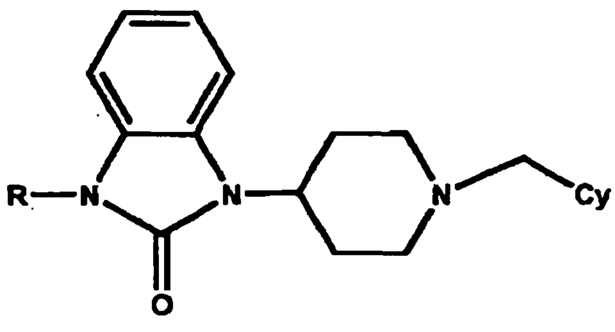

(I)

dans lequel R représente un groupe alkyle $C_1$-$C_6$ substitué dihydroxy et Cy est un groupe spiro[4.5]déc-6-yl, spiro[2.5]oct-4-yl, spiro[3.5]non-5-yl, 3,3-diméthylbicyclo[2.2.1]hept-2-yl ou 1-spiro(bicyclo[2.2.1]heptane-2,1'cyclopropan)-3-yle, ou un sel pharmaceutiquement acceptable de celui-ci.

**2.** Le composé selon la revendication 1, dans lequel R est un groupe alkyle $C_3$-$C_4$ substitué dihydroxyle.

**3.** Le composé selon la revendication 2, dans lequel R est 2,3-dihydroxypropyle, 2-hydroxy-1-(hydroxyméthyl)éthyle ou 2,3-dihydroxy-2-méthylpropyle.

**4.** Le composé selon la revendication 1, dans lequel le composé représenté par la formule (I) est sélectionné parmi

le groupe consistant en:

1) 1-(2,3-dihydroxypropyl)-3-[1-(spiro[4.5]déc-6-ylméthyl)-pipéridin-4-yl]-1,3-dihydro-2H-benzimidazol-2-one,
2) 1-(2,3-dihydroxypropyl)-3-[1-(spiro[2.5]oct-4-ylméthyl)-pipéridin-4-yl]-1,3-dihydro-2H-benzimidazol-2-one,
3) 1-[2-hydroxy-1-(hydroxyméthyl)éthyl]-3-[1-(spiro[bicyclo-[2.2.1]heptane-2, 1'-cyclopropan]-3-ylméthyl)pipéridin-4-yl]-1,3-dihydro-2H-benzimidazol-2-one,
4) 1-(2,3-dihydroxy-2-méthylpropyl)-3-[1-(spiro[2.5]oct-4-ylméthyl)-pipéridin-4-yl]-1,3-dihydro-2H-benzimidazol-2-one, et
5) 1-(2,3-dihydroxypropyl)-3-[1-(spiro[bicyclo[2.2.1]heptane-2,1'-cyclopropan]3-ylméthyl)pipéridin-4-yl]-1,3-dihydro-2H-benzimidazol-2-one, ou un sel pharmaceutiquement acceptable de ceux-ci.

**5.** Le composé selon la revendication 1, dans lequel le composé représenté par la formule (I) est du 1-(2,3-dihydroxypropyl)-3-[1-(spiro[4.5]déc-6-ylméthyl)-pipéridin-4-yl]-1,3-dihydro-2H-benzimidazol-2-one, ou un sel pharmaceutiquement acceptable de celui-ci.

**6.** Le composé selon la revendication 1, dans lequel le composé représenté par la formule (I) est du 1-(2,3-dihydroxypropyl)-3-[1-(spiro[2.5]oct-4-ylméthyl)-pipéridin-4-yl]-1,3-dihydro-2H-benzimidazol-2-one, ou un sel pharmaceutiquement acceptable de celui-ci.

**7.** Le composé selon la revendication 1, dans lequel le composé représenté par la formule (I) est du 1-[2-hydroxy-1-(hydroxyméthyl)éthyl]-3-[1-(spiro[bicyclo-[2.2.1]heptane-2,1'-cyclopropan]-3-ylméthyl)pipéridin-4-yl]-1,3-dihydro-2H-benzimidazol-2-one, ou un sel pharmaceutiquement acceptable de celui-ci.

**8.** Le composé selon la revendication 1, dans lequel le composé représenté par la formule (I) est du 1-(2,3-dihydroxy-2-méthylpropyl)-3-[1-(spiro[2.5]oct-4-ylméthyl)-pipéridin-4-yl]-1,3-dihydro-2H-benzimidazol-2-one, ou un sel pharmaceutiquement acceptable de celui-ci.

**9.** Le composé selon la revendication 1, dans lequel le composé représenté par la formule (I) est du 1-(2,3-dihydroxypropyl)-3-[1-(spiro[bicyclo[2.2.1]heptane-2,1'-cyclopropan]3-ylméthyl)pipéridin-4-yl]-1,3-dihydro-2H-benzimidazol-2-one, ou un sel pharmaceutiquement acceptable de celui-ci.

**10.** Composition pharmaceutique comprenant un adjuvant pharmaceutiquement acceptable et un composé selon l'une quelconque des revendications 1-9 ou un sel pharmaceutiquement acceptable de celui-ci.

**11.** Procédé de fabrication d'un médicament à utiliser en qualité d'analgésique, d'agent de soulagement contre une tolérance aux analgésiques narcotiques; d'agent de soulagement contre la dépendance aux analgésiques narcotiques ou contre une dépendance; d'agent renforceur analgésique, d'agent anti-obésité ou suppresseur de l'appétit; d'agent contre une détérioration cognitive et la démence/amnésie dans le vieillissement, une maladie cérébrovasculaire et la maladie d'Alzheimer; d'agent pour le traitement d'une anomalie cognitive du développement dans le trouble de déficit de l'attention, hyperactivité et trouble de l'apprentissage; de remède contre la schizophrénie; d'agent pour le traitement de maladies neurodégénératives représentées par le parkinsonisme et la chorée; d'agent antidépresseur ou pour le traitement d'un trouble affectif; d'agent de traitement ou prophylactique dans le diabète insipide; d'agent de traitement ou prophylactique dans la polyurie et de remède pour hypotension; lequel comprend combiner un composé selon l'une quelconque des revendications 1-9 ou un sel pharmaceutiquement acceptable de celui-ci et un adjuvant pharmaceutiquement acceptable.

**12.** Composé selon l'une quelconque des revendications 1-9 à utiliser en médecine.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- WO 9854168 A
- WO 0139775 A

### Non-patent literature cited in the description

- *Nature,* 1995, vol. 377, 532
- *Society for Neuroscience,* 1996, vol. 22, 455
- *NeuroReport,* 1997, vol. 8, 423
- *Eur. J. Neuroscience,* 1997, vol. 9, 194
- *Neuroscience,* 1996, vol. 75, 1
- *NEUROSCIENCE,* 1996, 333
- *Life Sciences,* 1997, vol. 60, PL15
- *LIFE SCIENCES,* 1997, PL141
- *Proceedings for National Academy of Sciences,* 1997, vol. 94, 14858
- *Neuroscience Letters,* 1997, vol. 237, 136
- *Nature,* 1998, vol. 394, 577
- *Psychopharmacology,* 2000, vol. 151, 344-350
- *Journal of Neuroscience,* 2000, vol. 20, 7640
- *Proceedings for National Academy of Sciences,* 1999, vol. 96, 10444
- *J. Med Chem.,* 1999, 5061-5063
- *J. Med. Chem.,* 1999, 5061-5063
- **T. W. Greene.** Protective Groups in Organic Synthesis. John Wiley & Sons Co, 1981